# EUROPEAN PATENT SPECIFICATION

(11) **EP 2 010 565 B1**
(45) Date of publication and mention of the grant of the patent: **22.02.2012**
(21) Application number: 07748645.4
(22) Date of filing: 16.04.2007
(51) Int. Cl.: C07K 14/715, C12N 15/85, C12N 5/16

(54) **METHOD FOR RECOMBINANT PROTEIN PRODUCTION IN CHO CELLS**
VERFAHREN ZUR PRODUKTION VON REKOMBINANTEM PROTEIN IN CHO-ZELLEN
PROCÉDÉ DE PRODUCTION DE PROTÉINE RECOMBINANTE DANS DES CELLULES CHO

(30) Priority: 21.04.2006 US 745289 P
(43) Date of publication of application: 07.01.2009
(73) Proprietor: Agency for Science, Technology and Research, Singapore 138632 (SG)
(72) Inventor: LEE, Yih Yean, Singapore 138668 (SG); WONG, Kathy, Singapore 138668 (SG)
(74) Representative: Viering, Jentschura & Partner
(86) International application number: PCT/SG2007/000100
(87) International publication number: WO 2007/123489

(56) References cited:
- WO-A-2005/010046
- US-A1- 2004 157 289
- LEE YONG J ET AL: "Alteration of heat sensitivity by introduction of HSP70 or anti-HSP70 antibody in CHO cells" JOURNAL OF THERMAL BIOLOGY, vol. 18, no. 4, 1993, pages 229-236, XP002449657 ISSN: 0306-4565
- HUOT J ET AL: "INCREASED SURVIVAL AFTER TREATMENTS WITH ANTICANCER AGENTS OF CHINESE HAMSTER CELLS EXPRESSING THE HUMAN M-R 27000 HEAT SHOCK PROTEIN" CANCER RESEARCH, vol. 51, no. 19, 1991, pages 5245-5252, XP002449658 ISSN: 0008-5472
- LAVOIE J ET AL: "SEQUENCE OF THE CHINESE HAMSTER SMALL HEAT SHOCK PROTEIN HSP27" NUCLEIC ACIDS RESEARCH, vol. 18, no. 6, 1990, page 1637, XP002449659 ISSN: 0305-1048 & DATABASE UniProt [Online] 1 April 1990 (1990-04-01), "Heat shock protein beta-1 (HspB1) (Heat shock 27 kDa protein) (HSP 27)." retrieved from EBI accession no. UNIPROT:P15991 Database accession no. P15991
- LANDRY J ET AL: "HEAT SHOCK RESISTANCE CONFERRED BY EXPRESSION OF THE HUMAN HSP27 GENE IN RODENT CELLS" JOURNAL OF CELL BIOLOGY, vol. 109, no. 1, 1989, pages 7-16, XP002449660 ISSN: 0021-9525
- GAESTEL MATTHIAS ET AL: "Structure and organisation of a murine gene encoding small heat-shock protein Hsp25" GENE (AMSTERDAM), vol. 128, no. 2, 1993, pages 279-283, XP002449661 ISSN: 0378-1119 & DATABASE UniProt [Online] 1 April 1990 (1990-04-01), "Heat shock protein beta-1 (HspB1) (Heat shock 27 kDa protein) (HSP 27) (Growth-related 25 kDa protein) (P25) (HSP25)." retrieved from EBI accession no. UNIPROT:P14602 Database accession no. P14602
- HE H ET AL: "HSP70 and heat shock factor 1 cooperate to repress Ras-induced transcriptional activation of the c-fos gene." CELL STRESS & CHAPERONES NOV 2000, vol. 5, no. 5, November 2000 (2000-11), pages 406-411, XP002449707 ISSN: 1355-8145

## Description

### FIELD

This invention relates to the fields of biotechnology and molecular biology. The invention particularly relates to novel genes from Chinese hamster, *Cricetulus griseus,* and the use of these genes for enhancing recombinant protein production in CHO cells.

### BACKGROUND

Lee *et al* 1993 discloses alteration of heat sensitivity by introduction of HSP70 or anti-HSP70 antibody in CHO cells.

US2004/157289 discloses use of small heat shock proteins such as HSP27.

Huot *et al* 1991 discloses increased survival after treatments with anticancer agents of chinese hamster cells expressing the human M-R 27000 heat shock protein.

Lavoie *et al* 1990 discloses sequence of the Chinese hamster small heat shock protein HSP27.

Landry *et al* 1989 discloses heat shock resistance conferred by expression of the human HSP27 gene in rodent cells.

WO2005/010046 discloses the use of cells expressing HSP70.

Gaestel *et al* 1993 discloses the structure and organisation of a murine gene encoding small heat shock protein Hsp25.

He *et al* 2000 discloses that HSP70 and heat shock factor 1 cooperate to repress Ras-induced transcriptional activation of the c-fos gene.

### SUMMARY

Our invention is based on the demonstration that over-expression of heat shock proteins, for example, HSP27 and HSP70, in CHO cells results in improved culture viability, extended culture time and improved protein yield.

Thus the invention provides a method of culturing a Chinese Hamster Ovary (CHO) cell in a bioreactor, the method comprising fed-batch culture of a CHO cell which is engineered, or which an ancestor thereof has been engineered, to up-regulate the expression of HSP27 comprising the sequence set out as SEQ ID NO: 2 or a sequence having at least 98.5% sequence identity thereto, in which up-regulation of expression of HSP27 results in an increase in integrated viable cell density (IVCD) of at least 50%, compared to culture of CHO cells which have not been so engineered.

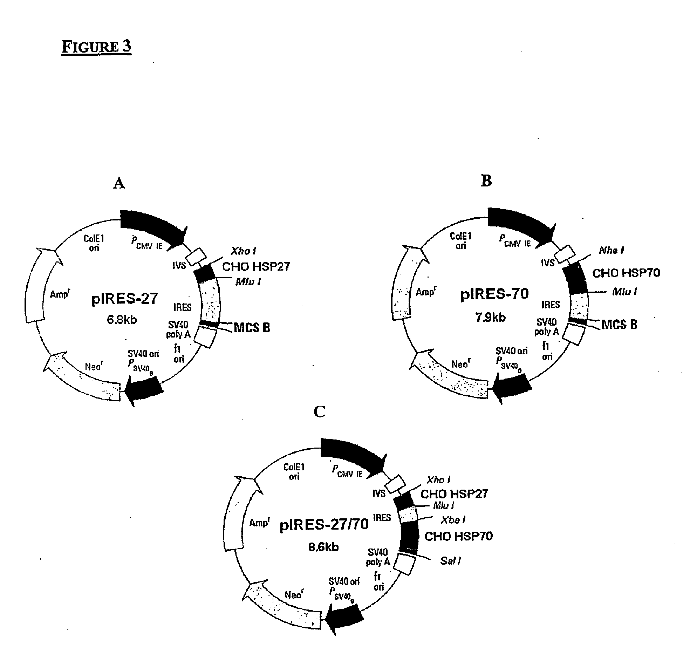

Preferably the method comprises introducing an expression vector capable of expressing HSP27, such as pIRES-27 (Figure 3A), into the CHO cell or an ancestor thereof.

Preferably the CHO cell is a stable cell line capable of over-expressing HSP27.

Preferably the CHO cell displays any one or more of the following: (a) an extension of culture time, such as by between 36 and 72 hours: (b) a delayed or reduced apoptosis; (c) a higher viability; (d) a slower rate of viability loss; (e) a reduced or delayed (such as by between 24 to 48 hours) expression of an apoptotic marker, such as caspase 2, caspase 3, caspase 8 or caspase 9; (f) an increase in integrated viable cell density (IVCD) of at least 75%, at least 100% or at least 120%; (g) increased maximum cell concentration in culture, or an extension in culture lifespan, or both; (h) enhanced recombinant protein expression, such as enhanced glycoprotein such as interferon-γ (IFN-γ) expression, such as by about 1.8x, about 2x, or about 2.8x; and (i) a higher viability; compared to a CHO cell in which the HSP27 and optionally HSP70 protein(s) is or are not up-regulated.

Preferably the CHO cell or ancestor thereof is engineered to up-regulate the expression of HSP70 comprising the sequence SEQ ID NO: 4 or a sequence having at least 90% sequence identity thereto.

Preferably the method comprises introducing an expression vector capable of expressing HSP70, such as pIRES-70 (Figure 3B) or pIRES-27/70 (Figure 3C) into the CHO cell or an ancestor thereof.

Preferably the CHO cell comprises an expression sequence capable of expressing a protein of interest, such as a glycoprotein such as interferon-γ (IFN-γ) or an antibody such as a monoclonal antibody, for example, a humanized IgG1 monoclonal antibody against Rhesus(D)-antigen.

The invention also relates to a method of expressing a recombinant protein comprising a method as described above and expressing a recombinant protein from the CHO cell.

Preferably the recombinant protein comprises a glycoprotein such as interferon-γ (IFN-γ) or an antibody such as a monoclonal antibody, for example, a humanized IgG1 monoclonal antibody against Rhesus(D)-antigen.

Preferably the method as described above is for providing a fed-batch culture of Chinese Hamster Ovary (CHO) cells with extended lifespan in a bioreactor.

In another aspect, the invention relates to a method of enhancing expression of Interferon-γ (IFN-γ) or an antibody such as a monoclonal antibody, for example, a humanized IgG1 monoclonal antibody against Rhesus(D)-antigen, by a Chinese Hamster Ovary (CHO) cell in culture, such as by an increase of yield of about 1.8x, about 2x, or about 2.8x, the method comprising a method as described above.

Preferably, the cell is a Chinese Hamster Ovary (CHO) cell, preferably a CHO-KI cell (ATCC CCL-61).

Preferably, the cell is a CHO-IFN-γ cell.

Preferably, the cell is engineered by introducing an expression vector capable of expressing the heat shock protein into the cell or an ancestor thereof.

Preferably, the expression vector is selected from the group consisting of: pIRES- 27 (Figure 3A), pIRES-70 (Figure 3B) and pIRES-27/70 (Figure 3C).

Preferably, the cell is a stable cell line capable of over-expressing the heat shock protein.

Preferably, the cell expresses a higher amount of heat shock protein HSP27.

Preferably, the HSP27 comprises the sequence SEQ ID NO: 2, or is encoded by the sequence SEQ ID NO: 1.

Preferably, the cell expresses a higher amount of heat shock protein HSP70.

Preferably, the HSP70 comprises the sequence SEQ ID NO: 4, or is encoded by the sequence SEQ ID NO: 3.

Preferably, the cell expresses a higher amount of both HSP27 and HSP70.

Preferably, the engineered cell displays an extension of culture time compared to a cell which is hot engineered.

Preferably, the culture time is extended by between 36 and 72 hours.

Preferably, the engineered cell displays a delayed or reduced apoptosis compared to a cell which is not engineered.

Preferably, the engineered cell displays a higher viability compared to a cell which is not engineered.

Preferably, the engineered cell displays a slower rate of viability loss compared to a cell which is not engineered.

Preferably, the engineered cell displays a delayed or reduced expression of an apoptotic marker compared to a cell which is not engineered.

Preferably, the apoptotic marker is selected from the group consisting of: caspase 2, caspase 3, caspase 8 and caspase 9.

Preferably, expression of the apoptotic marker is delayed by between 24 to 48 hours.

Preferably, the engineered cell displays an increase in integrated cell viable density (ICVD) compared to a cell which is not engineered.

Preferably, the integrated cell viable density (ICVD) is increased by at least 50%, preferably at least 75%, more preferably at least 100%, most preferably at least 120%.

Preferably, the ICVD increase results from an increase in the maximum cell concentration attained in culture, or an extension in culture lifespan, or both.

Preferably, the engineered cell is capable of enhanced recombinant protein expression compared to a cell which is not engineered.

Preferably, the recombinant protein is a glycoprotein, preferably interferon-γ.

Preferably, the yield of expressed recombinant interferon-γ is increased by about 1.8x, preferably about 2x, more preferably about 2.8x.

Preferably, the recombinant protein is a glycoprotein, preferably interferon-γ.

Preferably, the yield of expressed recombinant interferon-γ is increased by about 1.8x, preferably about 2x, more preferably about 2.8x, compared to the yield from a cell which is not so engineered.

Preferably, the method is conducted in a bioreactor, preferably in a batch or fed-batch method.

Also described is an expression sequence, preferably an expression vector, comprising a such polynucleotide according or a portion thereof operably linked to a regulatory sequence, the regulatory sequence capable of directing expression of said polynucleotide.

Preferably, the expression sequence is pIRES-27 (Figure 3A)

Preferably, the expression sequence is pIRES-70 (Figure 3B)

Preferably, the expression sequence is prRES-27/70 (Figure 3C).

Also described is a method of producing a Polypeptide comprising: (a) providing such an expression sequence and a regulatory sequence, in which the regulatory sequence is capable of directing expression of the polypeptide from the polynucleotide sequence, (b) allowing expression of the polypeptide from the expression sequence under control of the regulatory sequence, and (c) optionally purifying the polypeptide. Also described is a method comprising modulating, preferably up-regulating, the expression of a HSP27 polypeptide having a sequence shown as SEQ ID NO: 2 or a HSP27 polynucleotide having a sequence shown as SEQ ID NO: 1 in a cell, preferably a *Cricetulus griseus* cell.

Also described is a method comprising modulating, preferably up-regulating, the expression of a HSP70 polypeptide having a sequence shown as SEQ ID NO: 4 or a HSP70 polynucleotide having a sequence shown as SEQ ID NO: 3 in a cell, preferably a *Cricetulus griseus* cell.

Preferably we provide such a method for: (a) extending the culture time of a cell or a cell line comprising the cell, preferably by between 36 and 72 hours; (b) delaying or reducing apoptosis in the cell or a cell line comprising the cell; (c) increasing the viability of the cell or a cell line comprising the cell; (d) slowing the rate of loss of viability of a cell or a cell line comprising the cell; (e) delaying or reducing the expression of an apoptotic marker, preferably caspase 2, caspase 3, caspase 8 and caspase 9, in a cell or a cell line comprising the cell, preferably by between 24 to 48 hours; (f) increasing the integrated cell viable density (ICVD) of a cell line comprising the cell, preferably by at least 50%, preferably at least 75%, more preferably at least 100%, most preferably at least 120%; (g) increasing the maximum cell concentration attained in culture, or an extension in culture lifespan, or both, of a cell line comprising the cell; (h) enhancing the expression of a recombinant protein, preferably glycoprotein, more preferably interferon-γ, by a cell or a cell line comprising the cell, preferably by about 1.8x, preferably about 2x, more preferably about 2.8x.

Further particular and preferred aspects of the present invention are set out in the accompanying independent and dependent claims. Features of the dependent claims may be combined with features of the independent claims as appropriate, and in combinations other than those explicitly set out in the claims.

The practice of the present invention will employ, unless otherwise indicated, conventional techniques of chemistry, molecular biology, microbiology, recombinant DNA and immunology, which are within the capabilities of a person of ordinary skill in the art. Such techniques are explained in the literature. See, for example, J. Sambrook, E. F. Fritsch, and T. Maniatis, 1989, Molecular Cloning: A Laboratory Manual, Second Edition, Books 1-3, Cold Spring Harbor Laboratory Press; Ausubel, F. M. et al. (1995 and periodic supplements; Current Protocols in Molecular Biology, ch. 9, 13, and 16, John Wiley & Sons, New York, N.Y.); B. Roe, J. Crabtree, and A. Kahn, 1996, DNA Isolation and Sequencing: Essential Techniques, John Wiley & Sons; J. M. Polak and James O'D. McGee, 1990, In Situ Hybridization: Principles and Practice; Oxford University Press; M. J. Gait (Editor), 1984, Oligonucleotide Synthesis: A Practical Approach, Irl Press; D. M. J. Lilley and J. E. Dahlberg, 1992, Methods of Enzymology: DNA Structure Part A: Synthesis and Physical Analysis of DNA Methods in Enzymology, Academic Press; Using Antibodies : A Laboratory Manual : Portable Protocol NO. I by Edward Harlow, David Lane, Ed Harlow (1999, Cold Spring Harbor Laboratory Press, ISBN 0-87969-544-7); Antibodies : A Laboratory Manual by Ed Harlow (Editor), David Lane (Editor) (1988, Cold Spring Harbor Laboratory Press, ISBN 0-87969-314-2), 1855. Handbook of Drug Screening, edited by Ramakrishna Seethala, Prabhavathi B. Fernandes (2001, New York, NY, Marcel Dekker, ISBN 0-8247-0562-9); and Lab Ref: A Handbook of Recipes, Reagents, and Other Reference Tools for Use at the Bench, Edited Jane Roskams and Linda Rodgers, 2002, Cold Spring Harbor Laboratory, ISBN 0-87969-630-3. Each of these general texts is herein incorporated by reference.

The term "transformation" refers to the transfer of a nucleic acid fragment into the genome of a host cell, resulting in genetically stable inheritance. A "host cell" is a cell that has been transformed, or is capable of transformation, by an exogenous nucleic acid molecule. Host cells containing the transformed nucleic acid fragments are referred to as "transgenic" cells, and organisms comprising transgenic cells are referred to as "transgenic organisms".

"Transformed", "transduced", "transgenic", and "recombinant" refer to a host cell or organism into which a heterologous nucleic acid molecule has been introduced. The nucleic acid molecule can be stably integrated into the genome generally known in the art and are disclosed in Sambrook and Russell, *infra.* See also Innis *et al.* (1995); and Gelfand (1995); and Innis and Gelfand (1999). Known methods of PCR include, but are not limited to, methods using paired primers, nested primers, single specific primers, degenerate primers, gene-specific primers, vector-specific primers, partially mismatched primers, and the like. For example, "transformed," "transformant," and "transgenic" cells have been through the transformation process and contain a foreign gene integrated into their chromosome. The term "untransformed" refers to normal cells that have not been through the transformation process.

A "transgenic" organism is an organism having one or more cells that contain an expression vector.

"Genetically altered cells" denotes cells which have been modified by the introduction of recombinant or heterologous nucleic acids (*e.g*., one or more DNA constructs or their RNA counterparts) and further includes the progeny of such cells which retain part or all of such genetic modification.

### BRIEF DESCRIPTION OF THE FIGURES

The present invention will be described further, by way of example only, with reference to preferred embodiments thereof as illustrated in the accompanying drawings, in which:
Figure 1. A schematic diagram showing the strategy used to engineer CHO cells that overexpress HSP27 and/or HSP70.
Figure 2. Summary of the apoptotic pathways regulated by HSP 27 and HSP 70.
Figure 3. Vector constructs used for overexpressing HSP27 (A), HSP70 (B) and both HSP27 and HSP70 (C) in the CHO cell line.
Figure 4. Western blot from late exponential and stationary phases of fed-batch cultures showing: (A) higher expression of HSP27 in p27 compared to Control; (B) higher expression of HSP70 in p70 compared to Control; (C) higher expression of both HSP27 and HSP70 in p27/70 compared to Control.
Figure 5. Cell concentration and viability profiles of (A) p27 fed-batch culture, (B) p70 fed-batch culture and (C) p27/70 fed-batch culture shows improvements in both cell growth and culture duration over Control (pBlank) fed-batch culture.
Figure 6. Caspases 3, 9, 8 and 2 activity profiles of p27 (■), p70 (◆), p27/70 (▲) and Control (broken line) indicates delays in caspases activation in all three engineered cell lines compared to Control.
Figure 7. Cumulative integrated viable cell density (IVCD) and interferon-γ concentration profiles of (A) p27 fed-batch culture, (B) p70 fed-batch culture and (C) p27/70 fed-batch culture shows improvements in both IVCD and culture production yield over Control (pBlank) fed-batch culture.

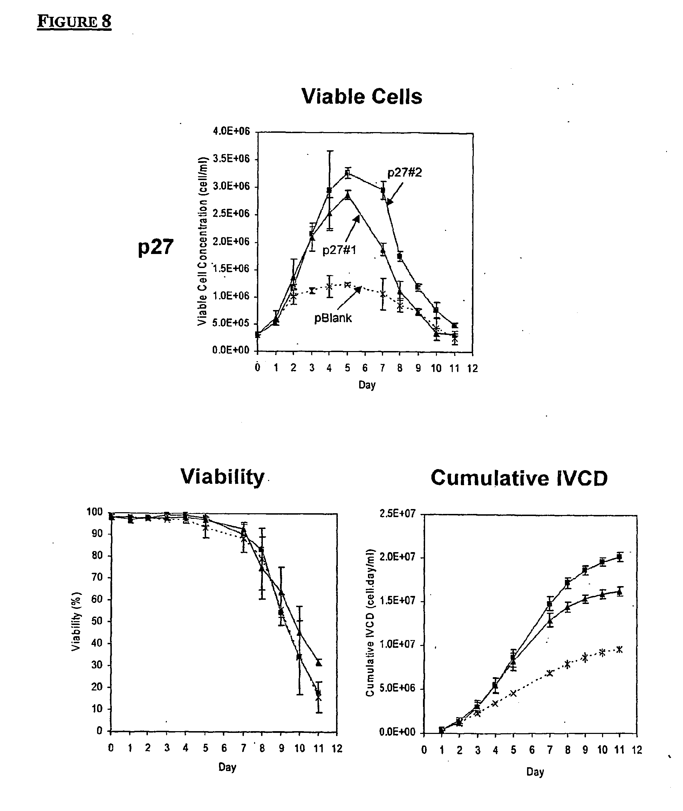

Figure 8. Response to stress induced by serum withdrawal. The number of viable cells, viability and cumulative IVCD is shown for CHO-K1 cells transfected in 10% FBS and single cell cloned. Stress is introduced by serum withdrawal. Retardation of growth is observed in control cells (pBlank), but p27 clones show no adverse effect due to serum withdrawal.

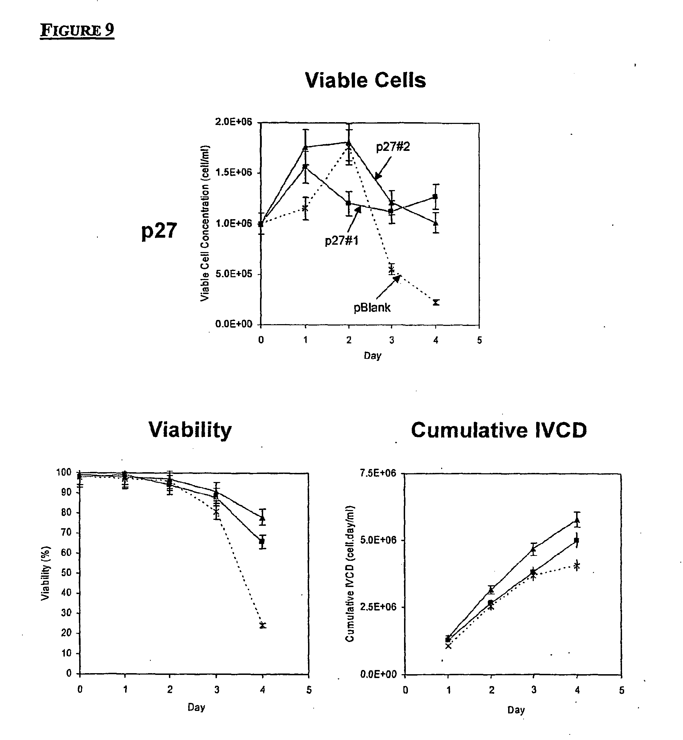

Figure 9. Response to stress induced by exposure to sodium butyrate. The number of viable cells, viability and cumulative IVCD is shown for CHO-K1 cells exposed to stress in the form of sodium butyrate. Control cells (pBlank) show a higher rate of decrease of viability compared to p27 clones. p27 clones exhibit significantly higher viabilities after 4 days.

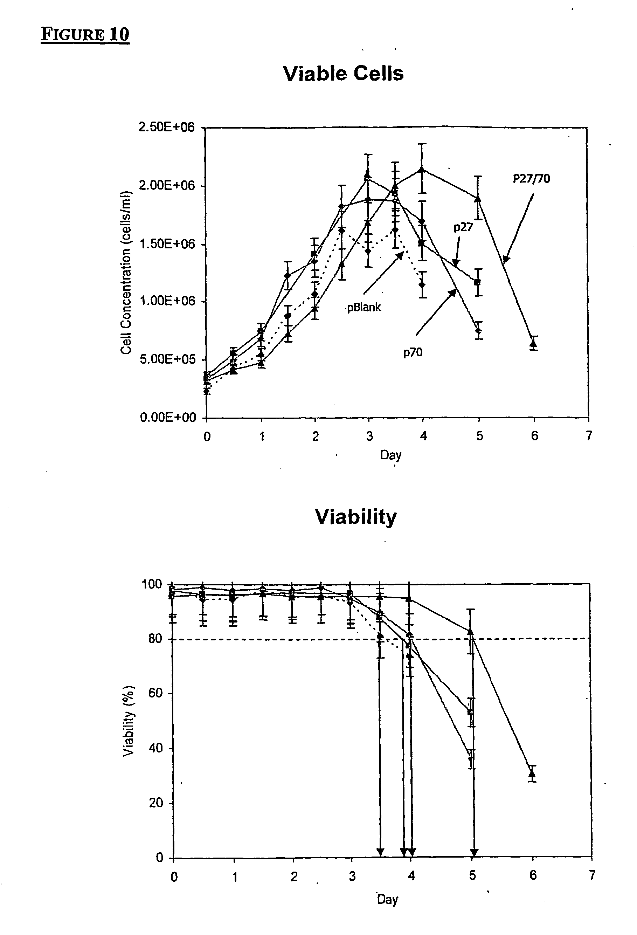

Figure 10. Viability of CHO-IFN-γ bioreactor batch cultures. The number of viable cells and viability is shown for CHO-IFN-γ cells transfected with HSP27, HSP70 and HSP27/HSP70 expression constructs. p27, p70 and p27/70 all show improved growth over pBlank (control). p27/70 exhibits a longer extension in viability than p27 or p70 over pBlank (eg. 36 hours vs 12 hours at 80% viability).

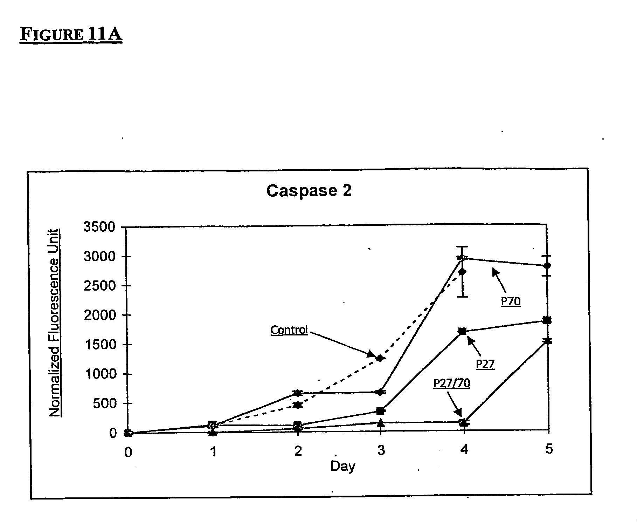

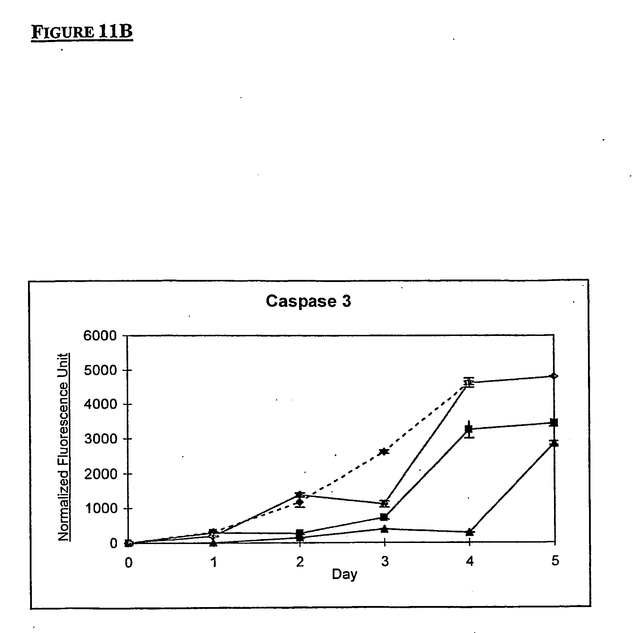

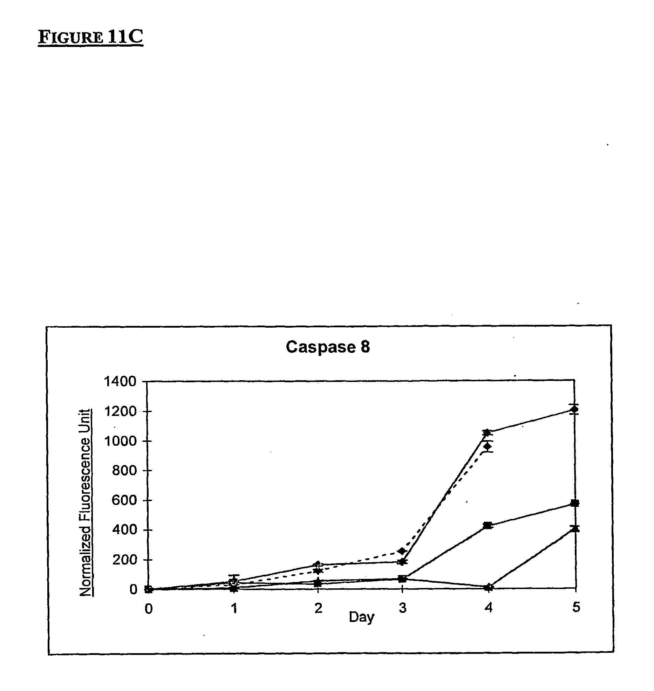

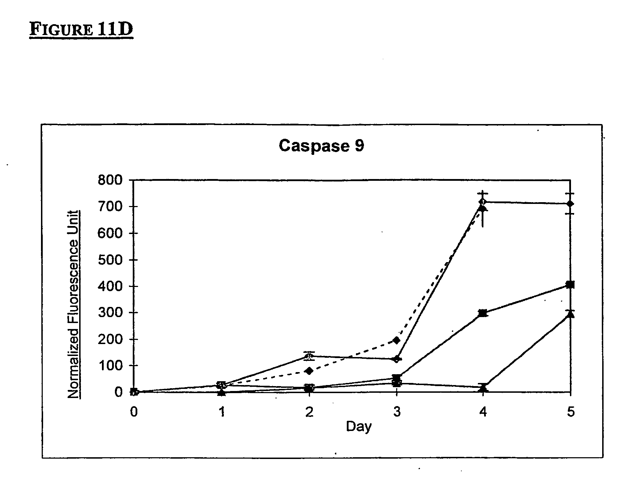

Figure 11. Caspase expression in CHO-IFN-γ bioreactor batch cultures. Expression of caspase 2, caspase 3, caspase 8 and caspase 9 are shown for CHO-IFN-γ cells transfected with HSP27, HSP70 and HSP27/HSP70 expression constructs. Figure 11A: caspase 2, Figure 11B: caspase 3, Figure 11C: caspase 8, Figure 11D: caspase 9. p27/70 delays caspases 3, 9, 8 and 2 activation longer than p27 when compared to pBlank (control). p70 shows similar caspases activation activities as pBlank.

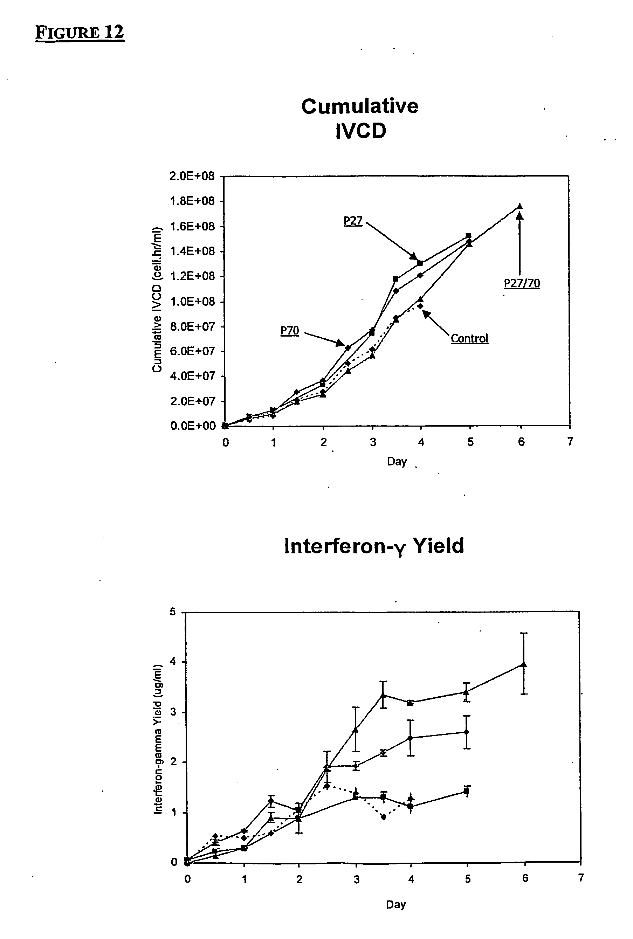

Figure 12. Interferon-γ production in CHO-IFN-γ bioreactor batch cultures. Cumulative IVCD and Interferon-γ yield are shown for CHO-IFN-γ cells transfected with HSP27, HSP70 and HSP27/HSP70 expression constructs. p27, p70 and p27/70 all show improvement in integrated viable cell density (IVCD) of between 50-80% over pBlank. p27/70 show 2.5-fold improvement in interferon-γ production over pBlank reaching 4 ug/ml. p70 shows a 70% improvement, however p27 shows similar production to pBlank.
Figure 13. Bicistronic expression vectors for over-expression of HSP27 and HSP70 in CHO-mAb cell line.
Figure 14. Experimental workflow for evaluation of HSP27 and HSP70 over-expression in CHO-mAb cell line.

### SEQUENCE LIST

**SEQ ID NO: 1** shows the CHO HSP27 Nucleotide Sequence (cgHSP27, 631 bp). **SEQ ID NO: 2** shows the CHO HSP27 Amino Acid Sequence (cgHSP27, 203 amino acids). **SEQ ID NO: 3** shows the CHO HSP70 Nucleotide Sequence (cgHSP70,1749 bp). **SEQ ID NO: 4** shows the CHO HSP70 Amino Acid Sequence (cgHSP70, 582 amino acids).

The methods and compositions described here may suitably employ any one or more of the sequences shown in the Sequence Listing.

### DETAILED DESCRIPTION

Our invention is based on the demonstration that over-expression of heat shock proteins, for example, HSP27 and HSP70, in CHO cells results in improved culture viability, extended culture time and improved protein yield.

As shown in the Examples, CHO-IFN-γ cells expressing recombinant human interferon-γ (IFN-γ) are engineered to overexpress two molecular chaperones HSP27 and HSP70 either individually or in combination. Batch and fed-batch bioreactor cultures are conducted and compared with control cultures utilizing cells transfected with the vector backbone. The Examples show that in batch and fed-batch cultures, the engineered cell lines exhibited a more gradual viability loss and extension of culture times of 36 to 72 hours.

These delays in viability decline are verified by corresponding delays in cellular caspases activation. Thus, we observe corresponding delays in escalation of caspases 2, 3, 8 and 9 activities, compared to the control cultures. These culture improvements also translate to an increase in recombinant protein yields. Thus, we find that the extension in culture times translates to a 2.5-fold improvement in IFN-γ production over controls in both batch and fed-batch cultures.

The improvements in culture growth and extended culture times are observed in cells overexpressing either or both HSPs.

We believe that the pleiotropic role of these HSPs as molecular chaperones to facilitate proper protein folding is an additional benefit (Mosser *et al.* 1994), since it improves protein secretion and even quality.

Accordingly, our invention provides a cellular engineering strategy which allows for development of stable robust cell lines that are resistant to a range of apoptotic insults and possess enhanced culture characteristics that improves recombinant protein yields. Such cell lines are suitable for the expression of any recombinant protein as known in the art.

### HEAT SHOCK PROTEINS

The engineered Chinese Hamster cell may express a higher level of any heat shock protein as known in the art. Examples include HSP100, HSP90, HSP70, HSP60 and the small heat-shock proteins (sHSPs).

Preferably, the heat shock protein comprises HSP27 or HSP70. In preferred embodiments, the HSP27 and HSP70 comprise the novel *Cricetulus griseus* heat shock protein sequences disclosed herein, namely, cgHSP27 and cgHSP70. The HSP27 may preferably comprise SEQ ID NO: 2 and the HSP70 may preferably comprise SEQ ID NO: 4, or a fragment, homologue, variant or derivative thereof.

We provide for engineered Chinese Hamster cells that over-express HSP27 as well as engineered Chinese Hamster cells that over-express HSP70. In a preferred embodiment, we provide for engineered Chinese Hamster cells that over-express HSP27 as well as HSP70. Specifically, we describe a CHO cell that expresses a higher level of both HSP27 and HSP70, compared to a cell which is not so engineered.

### RECOMBINANT PROTEIN EXPRESSION

The methods and compositions described here involve the use of a a *Cricetulus griseus* cell which is modified, preferably genetically engineered, so that the expression of a heat shock protein is up-regulated compared to a cell which has not been so modified. Such an engineered cell may be produced using the methods described in further detail below.

The *Cricetulus griseus* cell is preferably a Chinese Hamster Ovary (CHO) cell, preferably a CHO-K1 cell (ATCC CCL-61). In preferred embodiments, the cell comprises a CHO-IFN-γ cell.

The engineered cell may be used to produce any protein of interest, preferably at enhanced levels or higher yield, by comprising an expression sequence for a protein of interest. The expression sequence may suitably comprise an expression vector, in which a coding sequence for the protein of interest is operably linked to a regulatory sequence, which regulatory sequence is chosen so that expression therefrom is constitutive or inducible, as known in the art.

The engineered cell may be transformed or transfected with the expression sequence, using means known in the art. The cell may be transiently transfected, or stably transfected, such that it expresses cgHSP27 and/or cgHSP70 in a stable manner. The cell may suitably be in the form of a cell line or cell culture.

The protein of interest may comprise include those that are of therapeutic and/or diagnostic application such as, but not limited to: sequences encoding cytokines, chemokines, hormones, antibodies, anti-oxidant molecules, engineered immunoglobulin-like molecules, a single chain antibody, a humanised antibody, fusion proteins, enzymes, immune co-stimulatory molecules, immunomodulatory molecules, anti-sense RNA, a transdominant negative mutant of a target protein, a toxin, a conditional toxin, an antigen, a tumour suppresser protein and growth factors, membrane proteins, vasoactive proteins and peptides, anti-viral proteins and ribozymes, and derivatives thereof (such as with an associated reporter group). The protein of interest may also comprise pro-drug activating enzymes.

The protein of interest may be an interferon. The interferon may be interferon-γ (IFN-γ).

The protein of interest may be an antibody. The antibody may be a monoclonal antibody. It may comprise a humanized monoclonal antibody. It may comprise a IgG1 antibody. It may be against Rhesus(D)-antigen. Preferably, it may be a humanized IgG1 monoclonal antibody against Rhesus(D)-antigen.

In preferred embodiments, the protein of interest comprises a glycoprotein, or any other protein which has one or more post-translational modifications. For example, any protein which is suitable for production in a eukaryotic host may be expressed using the methods and compositions described here. In highly preferred embodiments, the protein of interest comprises interferon-γ.

### USES OF CGHSP27 AND CGHSP70 SEQUENCES

We disclose the sequence of novel *Cricetulus griseus* heat shock protein sequences, cgHSP27 and cgHSP70, which are described in further detail below. As shown in the Examples, we have established that the cgHSP27 and cgHSP70 genes are involved in the mediation of apoptosis in the cell.

We also show that targeting of such genes by modulation of their activity results in reduction of apoptosis and hence improved cell viability. The genes and polypeptides and products thereof therefore have utility in a number of fields, for example in cell culture.

Thus, US Patent Number 6,586,206 describes the use of apoptosis inhibitors in the production of recombinant proteins using cultured host cells, with the effect of improved yield of the desired protein. Accordingly, the disclosure of the sequences of *Cricetulus griseus* cgHSP27 and cgHSP70 therefore enables the targetting of these genes in cell culture to enhance cell viability and promote enhanced yields of recombinant protein production. Specifically, cgHSP27 and cgHSP70 modified cells we describe here, preferably *Cricetulus griseus* cells, more preferably Chinese Hamster Ovary cells, may be suitably employed for production of recombinant proteins with improved yield.

### CGHSP27 AND CGHSP70 MODIFIED CELLS

According to the methods and compositions described here, modulation of any one or more of cgHSP27 and cgHSP70 in a cell improves cell viability of a population, preferably a *Cricetulus griseus* population. In particular, we show in the Examples that increasing of expression of either or both of cgHSP27 and cgHSP70, leads to improved cell viability. However, it will be appreciated that methods of regulation of any of these genes, including use of modulator entities such as agonists, may be employed in addition to, or as an alternative to, modulation of polypeptide expression.

Cells in which the expression of any one or more of these genes are modulated are referred to for convenience as "modified" cells - although it will be appreciated that these may not be physically modified themselves, but may be descendants of cells which have been modified. We specifically provide for cells in which cgHSP27 and/or cgHSP70 expression is up-regulated.

Such modified cells may be advantageously used as hosts for the production of any recombinant protein of interest. In some embodiments, the modified cells are in suspension culture.

In some embodiments, the modified cells are cultured in a batch culture. By batch culture we preferably mean a closed system culture of microorganisms where a limited number of generations are allowed to grow before all nutrients are used up. Such a closed system may contain conditions to enable cell culture growth, such as specific nutrient types, temperature, pressure, aeration, and other environmental conditions.

In other embodiments, the modified cells are cultured in a fed-batch culture. A fed-batch culture is understood to mean a method of culturing cells in which a solution of nutrients is added at intervals during culture. In fed-batch mode, usually little or no product is harvested until the end of the run.

In some embodiments, the modified cells are cultured in a biorector.

As shown in the Examples, the expression yield of the recombinant protein is higher compared to cells which have not been so modified.

The expression yield may be at least 1.1x than of cells which have not been so modified. Specifically, the expression yield may be at least 1.5x, 1.6x, 1.7x, 1.8x, 1.9x, 2x, 2.1x, 2.2x, 2.3x, 2.4x, 2.5x, 2.6x, 2.7x, 2.8x, 2.9x, 3x, 3.1x, 3.2x, 3.3x, 3.4x, 3.5x, 3.6x, 3.7x, 3.8x, 3.9x, 4x or more than of cells which have not been so modified. The expression yield may be at least 5x, 6x, 7x, 8x, 9x, 10x, 15x, 20x, 25x, 30x, 40x, 50x or more than of cells which have not been so modified.

The yield may be up to or more than 1 g/L of culture, preferably up to or more than 2 g/L, 3 g/L 4 g/L, 5 g/L, 6 g/L, 7 g/L, 8 g/L, 9 g/L, 10 g/L or more of culture.

We specifically disclose the production of a polypeptide of interest comprising providing an expression sequence comprising a sequence encoding the polypeptide of interest and a regulatory sequence, in which the regulatory sequence is capable of directing expression of the polypeptide from the polynucleotide sequence, allowing expression of the polypeptide from the expression sequence under control of the regulatory sequence in a host cell comprising a modified CHO cell as described here, and optionally purifying the polypeptide.

The relevant cells may be modified by targeting relevant genes by any means known in the art. In a preferred embodiment, the up-regulation of expression of cgHSP27 and/or cgHSP70 is achieved by introducing an expression sequence into the cell. The expression sequence preferably comprises a cgHSP27 and/or cgHSP70 polynucleotide or a portion thereof operably linked to a regulatory sequence, the regulatory sequence capable of directing expression of said polynucleotide. The expression sequence is suitably in the form of an expression vector, and preferred expression vectors include pIRES-27, pIRES-70 and pIRES-27/70. The structures of these vectors are shown respectively at Figure 3A, Figure 3B and Figure 3C, and methods of constructing them are shown in the Examples.

Preferably, the modified cells have at least 5%, preferably 10% or more, more preferably 15%, 20%, 30%, 40%, 50% or more viable cells compared to a control population. Alternatively, or in addition, the modified cells maintain cell viability for a longer period of time compared to cells which have not been modified. For example, modified cells are able to maintain a certain percentage cell viability (e.g., 95%) for a longer period compared to control cells.

Preferably, modified cells have extended cell viability by at least 1 hour, more preferably at least 6 hours, most preferably at least 12 hours or more, e.g., at least 24 hours, at least 36 hours or at least 48 hours, compared to control cells. In highly preferred embodiments, modified cells have extended viability by at least 24 hours before viability begins to drop below 95%, compared to control cells.

Preferably, cell viability is gauged by quantitating a viable cell density of a population of cells which have been modified, i.e., by targeting cgHSP27 or cgHSP70, or both. Preferably, the modified cells maintain a higher cell viability, compared to cells which have not been modified (e.g., a control population). Cell viability is preferably measured as the percentage of cells in the relevant cell population which are viable.

In a preferred embodiment, cell viability is determined by a "Trypan blue viability exclusion assay". This assay is commonly used for cell viability determination in the field of cell culture. A detailed protocol is set out in the Examples, but in brief: a cell suspension is mixed with 0.4% trypan blue in phosphate buffered solution and counted using a hemocytometer. Live cells appear round and refractile without any blue-dye coloration while dead cells absorb the dye and appear blue. Viability is then expressed as a percentage of viable cells over total cells counted.

A viable cell is defined as a cell that whose membrane integrity is still able to prevent the absorption of trypan blue in a trypan blue exclusion viability assay.

### CHINESE HAMSTER HEAT SHOCK PROTEIN SEQUENCES

The disclosure provides generally for certain nucleic acids, polypeptides, as well as fragments, homologues, variants and derivatives thereof from the Chinese hamster, *Cricetulus griseus,* which are heat shock proteins.

In particular, we provide for *Cricetulus griseus* cgHSP27 and cgHSP70 polypeptide and nucleic acid sequences as set out in the Sequence Listings. In addition we provide for the use of such genes, fragments, homologues.

### CRICETULUS GRISEUS CGHSP27 AND CGHSP70 POLYPEPTIDES

It will be understood that polypeptide sequences disclosed here are not limited to the particular sequences set forth in the sequence listing, or fragments thereof, or sequences obtained from *Cricetulus griseus* cgHSP27 and cgHSP70 protein, but also include homologous sequences obtained from any source, for example related cellular homologues, homologues from other species and variants or derivatives thereof, provided that they have at least one of the biological activities of cgHSP27 and cgHSP70, as the case may be.

This disclosure therefore encompasses variants, homologues or derivatives of the amino acid sequences set forth in the sequence listings, as well as variants, homologues or derivatives of the amino acid sequences encoded by the nucleotide sequences disclosed here. Such sequences are generally referred to as a "cgHSP27 sequence" or a "cgHSP70 sequence", as the case may be.

### Biological Activities

In highly preferred embodiments, the sequences comprise at least one biological activity of cgHSP27 and cgHSP70, as the case may be.

Preferably, the biological activity comprises heat shock protein or molecular chaperone activity. Alternatively, or in addition, the biological activity may comprise apoptosis inhibiting activity, preferably assayed by down-regulation of caspase activity. Thus, the cgHSP27 and cgHSP70sequences described in this document preferably are capable of inhibiting apoptosis, specifically capable of down-regulating caspase activity in the context of a cell.

In highly preferred embodiments, when assayed using such methods, the cgHSP27 and cgHSP70 sequences when transfected into a cell either alone or in combination are capable of inhibiting apoptosis by at least 10%, preferably 20%, more preferably 30%, 40% 50%, 60%, 70%, 80%, 90% or more, compared to a cell which has not been so transfected with the relevant cgHSP27 or cgHSP70 sequence.

In highly preferred embodiments, the repression of apoptosis by the cgHSP27 and/or cgHSP70sequences is assayed by assaying caspase activity. Thus, the percentage stimulation or repression of apoptosis set out above are in highly preferred embodiments to be read as percentage stimulation or repression of caspase activity.

Thus, apoptosis activity monitoring methods such as caspase activity measurement assays using colorimetric or fluorometric methods can be used to ascertain the biochemical activity of cgHSP27 and cgHSP70. Such methods may be carried out in cells transfected with appropriate expression constructs, such as by means known in the art, or using protocols set out in the Examples, to determine whether apoptosis is affected and/or caspase activity is up- or down-regulated.

Caspases are a large family of cysteine proteases that mediates apoptosis (Nicholson & Thornberry 1997; Thornberry & Littlewood 1998). Caspase-8 is an initiator caspase that act the most upstream in receptor-mediated apoptotic pathway. Upon activation of cell-surface receptors, caspase-8 directly or indirectly initiates the proteolytic activities of downstream effector caspases such as caspase-3 (Srinivasula *et al* 1996 and Cohen 1997). Caspase-9, which is also another upstream caspase, is activated via the mitochondrial release of cytochrome c to the cytosol. Released cytochrome c binds to the apoptotic protease activating factor, APAF-1, forming a complex that activates procaspase-9 (Zou *et al* 1999 and Hu *et al* 1999). Active caspase-9 initiates a protease cascade that also activates caspase-3 and other downstream caspases.

In preferred embodiments, the caspase activity that is assayed to determine up- or down-regulation of apoptosis activity comprises caspase-2, caspase-3, caspase-8 or caspase-9.

Methods for assaying caspase-8 and caspase-9 activity are known in the art, and are specifically described in, for example, Nicholson DW and Thornberry NA (1997) Caspases: killer proteases. Trends Biochem Sci. 272: 2952-2956 and Thornberry NA and Littlewood Y (1998) Caspases: Enemies within. Science 281:1312-1316. Any of the protocols set out in the prior art may be used to assay caspase activity.

In preferred embodiments, however, the "Caspase Assay Protocol" set out below is employed to assay caspase-8 and/or caspase-9 activity.

### Caspase Assay Protocol

Caspase activity can be assayed by utilizing fluorogenic substrates specific for different caspases immobilized in the wells. Application of cell lysates containing the active caspase to the wells will cleave the substrate and release a fluorescent product that can be detected using standard fluorescence plate reader.

Specifically, BD ApoAlert^{™} Caspase assay plates (catalogue number K2033-1, BD Biosciences Clontech, Palo Alto, California, USA) uses different caspase substrates composed of short peptides that are recognized by their respective activated caspases. The peptides are covalently linked to the fluorogenic dye 7-amino-4-methyl coumarin (AMC). Peptide bound AMC emits in the UV range (λₘₐₓ=380nm) while unbound AMC emits in the green range (λₘₐₓ=460nm). This makes it possible to correlate an increase in fluorescence intensity at 460nm with an increase in activity of the respective caspase in the test sample. For assaying caspase-8 activity, the substrate used is VDVAD-AMC while an assay for caspase-9 activity uses LEHD-AMC as its substrate.

In order to assay for caspases activity using BD ApoAlert^{™} Caspase assay plate, cells from samples are pelleted by centrifugation and then resuspended in 1x cell lysis buffer (BD Biosciences Clontech) and incubated on ice for 10 min. Cellular debris is then removed by centrifugation for 5 min at 4°C. 50µL of 2X reaction buffer/DTT mix is then added to each well of the 96-well plate that will be used. The plate is preincubated at 37°C for 5 min. 50µL of the appropriate cell lysate(s) is then added to the wells and incubated at 37°C for 2 hour. A fluorescence plate reader is then used to measure the amount of AMC released (Excitation at 380nm, Emission at 460nm).

Caspase activity is defined as the absolute emission at 460nm of a sample after subtraction from the absolute emission at 460nm of a reference sample. The reference sample is a sample collected at time reference zero.

Caspase activity of cells transfected with cgHSP27 and/or cgHSP70 expression vectors may be compared with cells transfected with null-vectors (or untransfected) to determine the percentage by which apoptosis is stimulated or repressed as the case may be.

### [End of "Caspase Assay Protocol"]

In preferred embodiments, when assayed using such methods, the cgHSP27 and cgHSP70 sequences when transfected into a cell either alone or in combination are capable of inhibiting the expression of caspase-8, or caspase-9, or both by at least 10%, preferably 20%, more preferably 30%, 40% 50%, 60%, 70%, 80%, 90% or more, compared to a cell which has not been so transfected with the relevant sequence.

Other assays that detect apoptosis related events such as membrane changes, DNA fragmentation and other biochemical hallmarks of apoptosis can also be used, instead of, or in addition to, the assays described.

### Homologues

The polypeptides disclosed include homologous sequences obtained from any source, for example related viral/bacterial proteins, cellular homologues and synthetic peptides, as well as variants or derivatives thereof. Thus polypeptides also include those encoding homologues of cgHSP27 and cgHSP70 from other species including animals such as mammals (e.g. mice, rats or rabbits), in particular rodents.

In the context of the present document, a homologous sequence or homologue is taken to include an amino acid sequence which is at least 60, 70, 80 or 90% identical, preferably at least 95 or 98% identical at the amino acid level over at least 30, preferably 50, 70, 90 or 100 amino acids with cgHSP27 or cgHSP70, as the case may be, for example as shown in the sequence listing herein. In the context of this document, a homologous sequence is taken to include an amino acid sequence which is at least 15, 20, 25, 30, 40, 50, 60, 70, 80 or 90% identical, preferably at least 95 or 98% identical at the amino acid level, preferably over at least 15, 25, 35, 50 or 100, preferably 200, 300, 400 or 500 amino acids with the sequence of cgHSP27 and cgHSP70. For example, a sequence may have the stated sequence identity to cgHSP27 (preferably comprising a sequence as shown in SEQ ID NO: 2) or cgHSP70 (preferably comprising a sequence as shown in SEQ ID NO: 4).

In preferred embodiments, a cgHSP27 polypeptide has at least 98.1% or more sequence identity to a sequence shown as SEQ ID NO: 2. Preferably, the cgHSP27 polypeptide has 98.2% or more, preferably 98.3% or more, 98.4% or more, 98.5% or more, 98.6% or more, 98.7% or more, 98.8% or more, 98.9% or more, 99.0% or more or 99.1% or more, 99.2% or more, preferably 99.3% or more, 99.4% or more, 99.5% or more, 99.6% or more, 99.7% or more, 99.8% or more, 99.9% or more sequence identity to a sequence shown as SEQ ID NO: 2.

Similarly, in preferred embodiments , a cgHSP70 polypeptide has at least 90.1% or more sequence identity to a sequence shown as SEQ ID NO: 4. Preferably, the cgHSP27 polypeptide has 91% or more, preferably 92% or more, 93% or more, 94% or more, 95% or more, 96% or more, 97% or more, 98% or more, 99% or more or 99.1% or more, 99.2% or more, preferably 99.3% or more, 99.4% or more, 99.5% or more, 99.6% or more, 99.7% or more, 99.8% or more, 99.9% or more sequence identity to a sequence shown as SEQ ID NO: 4.

Although homology can also be considered in terms of similarity (i.e. amino acid residues having similar chemical properties/functions), in the context of the present document it is preferred to express homology in terms of sequence identity. In highly preferred embodiments, the sequence identity is determined relative to the entirety of the length the relevant sequence, i.e., over the entire length or full length sequence of the relevant gene, for example.

Homology comparisons can be conducted by eye, or more usually, with the aid of readily available sequence comparison programs. These commercially available computer programs can calculate % homology between two or more sequences.

% homology may be calculated over contiguous sequences, i.e. one sequence is aligned with the other sequence and each amino acid in one sequence directly compared with the corresponding amino acid in the other sequence, one residue at a time. This is called an "ungapped" alignment. Typically, such ungapped alignments are performed only over a relatively short number of residues (for example less than 50 contiguous amino acids).

Although this is a very simple and consistent method, it fails to take into consideration that, for example, in an otherwise identical pair of sequences, one insertion or deletion will cause the following amino acid residues to be put out of alignment, thus potentially resulting in a large reduction in % homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids, a sequence alignment with as few gaps as possible - reflecting higher relatedness between the two compared sequences - will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons. For example when using the GCG Wisconsin Bestfit package (see below) the default, gap penalty for amino acid sequences is -12 for a gap and -4 for each extension.

Calculation of maximum % homology therefore firstly requires the production of an optimal alignment, taking into consideration gap penalties. A suitable computer program for carrying out such an alignment is the GCG Wisconsin Bestfit package (University of Wisconsin, U.S.A.; Devereux et al., 1984, Nucleic Acids Research 12:387). Examples of other software than can perform sequence comparisons include, but are not limited to, the BLAST package (see Ausubel *et al.,* 1999 *ibid* - Chapter 18), FASTA (Atschul et al., 1990, J. Mol. Biol., 403-410) and the GENEWORKS suite of comparison tools. Both BLAST and FASTA are available for offline and online searching (see Ausubel *et al.,* 1999 *ibid,* pages 7-58 to 7-60). However it is preferred to use the GCG Bestfit program.

Although the final % homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. GCG Wisconsin programs generally use either the public default values or a custom symbol comparison table if supplied (see user manual for further details). It is preferred to use the public default values for the GCG package, or in the case of other software, the default matrix, such as BLOSUM62.

Once the software has produced an optimal alignment, it is possible to calculate % homology, preferably % sequence identity. The software typically does this as part of the sequence comparison and generates a numerical result.

### Variants and Derivatives

The terms "variant" or "derivative" in relation to the amino acid sequences as described here includes any substitution of, variation of, modification of, replacement of, deletion of or addition of one (or more) amino acids from or to the sequence. Preferably, the resultant amino acid sequence retains substantially the same activity as the unmodified sequence, preferably having at least the same activity as the cgHSP27 and cgHSP70 polypeptides shown in the sequence listings. Thus, the key feature of the sequences - namely that they are capable of modulating one or more apoptotic processes - is preferably retained.

Polypeptides having the amino acid sequence shown in the Examples, or fragments or homologues thereof may be modified for use in the methods and compositions described here. Typically, modifications are made that maintain the biological activity of the sequence. Amino acid substitutions may be made, for example from 1, 2 or 3 to 10, 20 or 30 substitutions provided that the modified sequence retains the biological activity of the unmodified sequence. Amino acid substitutions may include the use of non-naturally occurring analogues, for example to increase blood plasma half-life of a therapeutically administered polypeptide.

Natural variants of cgHSP27 and cgHSP70 are likely to comprise conservative amino acid substitutions. Conservative substitutions may be defined, for example according to the Table below. Amino acids in the same block in the second column and preferably in the same line in the third column may be substituted for each other:

| | | |
|---|---|---|
| ALIPHATIC | Non-polar | G A P |
| | | I L V |
| | Polar - uncharged | C S T M |
| | | N Q |
| | Polar - charged | D E |
| | | K R |
| AROMATIC | | H F W Y |

### Fragments

Polypeptides disclosed here and useful as markers also include fragments of the above mentioned full length polypeptides and variants thereof, including fragments of the sequences set out in the sequence listings.

Polypeptides also include fragments of the full length sequence of any of the cgHSP27 and cgHSP70 polypeptides. Preferably fragments comprise at least one epitope. Methods of identifying epitopes are well known in the art. Fragments will typically comprise at least 6 amino acids, more preferably at least 10, 20, 30, 50 or 100 amino acids.

Included are fragments comprising, preferably consisting of, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 105, 110, 115, 120, 125, 130, 135, 140, 145 or 150, or more residues from a cgHSP27 and/or cgHSP70 amino acid sequence.

Polypeptide fragments of the cgHSP27 and cgHSP70 proteins and allelic and species variants thereof may contain one or more (e.g. 5, 10, 15, or 20) substitutions, deletions or insertions, including conserved substitutions. Where substitutions, deletion and/or insertions occur, for example in different species, preferably less than 50%, 40% or 20% of the amino acid residues depicted in the sequence listings are altered.

cgHSP27 and cgHSP70 and their fragments, homologues, variants and derivatives, may be made by recombinant means. However, they may also be made by synthetic means using techniques well known to skilled persons such as solid phase synthesis. The proteins may also be produced as fusion proteins, for example to aid in extraction and purification. Examples of fusion protein partners include glutathione-S-transferase (GST), 6xHis, GAL4 (DNA binding and/or transcriptional activation domains) and β-galactosidase. It may also be convenient to include a proteolytic cleavage site between the fusion protein partner and the protein sequence of interest to allow removal of fusion protein sequences. Preferably the fusion protein will not hinder the function of the protein of interest sequence. Proteins may also be obtained by purification of cell extracts from animal cells.

The cgHSP27 and cgHSP70 polypeptides, variants, homologues, fragments and derivatives disclosed here may be in a substantially isolated form. It will be understood that such polypeptides may be mixed with carriers or diluents which will not interfere with the intended purpose of the protein and still be regarded as substantially isolated. A cgHSP27 and/or cgHSP70 variant, homologue, fragment or derivative may also be in a substantially purified form, in which case it will generally comprise the protein in a preparation in which more than 90%, e.g. 95%, 98% or 99% of the protein in the preparation is a protein.

The cgHSP27 and cgHSP70 polypeptides, variants, homologues, fragments and derivatives disclosed here may be labelled with a revealing label. The revealing label may be any suitable label which allows the polypeptide , etc to be detected. Suitable labels include radioisotopes, e.g. ¹²⁵I, enzymes, antibodies, polynucleotides and linkers such as biotin. Labelled polypeptides may be used in diagnostic procedures such as immunoassays to determine the amount of a polypeptide in a sample. Polypeptides or labelled polypeptides may also be used in serological or cell-mediated immune assays for the detection of immune reactivity to said polypeptides in animals and humans using standard protocols.

cgHSP27 and cgHSP70 polypeptides, variants, homologues, fragments and derivatives disclosed here, optionally labelled, my also be fixed to a solid phase, for example the surface of an immunoassay well or dipstick. Such labelled and/or immobilised polypeptides may be packaged into kits in a suitable container along with suitable reagents, controls, instructions and the like. Such polypeptides and kits may be used in methods of detection of antibodies to the polypeptides or their allelic or species variants by immunoassay.

Immunoassay methods are well known in the art and will generally comprise: (a) providing a polypeptide comprising an epitope bindable by an antibody against said protein; (b) incubating a biological sample with said polypeptide under conditions which allow for the formation of an antibody-antigen complex; and (c) determining whether antibody-antigen complex comprising said polypeptide is formed.

The cgHSP27 and cgHSP70 polypeptides, variants, homologues, fragments and derivatives disclosed here may be used in *in vitro* or *in vivo* cell culture systems to study the role of their corresponding genes and homologues thereof in cell function, including their function in disease. For example, truncated or modified polypeptides may be introduced into a cell to disrupt the normal functions which occur in the cell. The polypeptides may be introduced into the cell by *in situ* expression of the polypeptide from a recombinant expression vector (see below). The expression vector optionally carries an inducible promoter to control the expression of the polypeptide.

The use of appropriate host cells, such as insect cells or mammalian cells, is expected to provide for such post-translational modifications (e.g. myristolation, glycosylation, truncation, lapidation and tyrosine, serine or threonine phosphorylation) as may be needed to confer optimal biological activity on recombinant expression products. Such cell culture systems in which the cgHSP27 and cgHSP70 polypeptides, variants, homologues, fragments and derivatives disclosed here are expressed may be used in assay systems to identify candidate substances which interfere with or enhance the functions of the polypeptides in the cell.

### CRICETULUS GRISEUS CGHSP27 AND CGHSP70 NUCLEIC ACIDS

We provide generally for a number of cgHSP27 and cgHSP70 nucleic acids, together with fragments, homologues, variants and derivatives thereof. These nucleic acid sequences preferably encode the polypeptide sequences disclosed here, and particularly in the sequence listings.

Preferably, the polynucleotides comprise cgHSP27 and cgHSP70 nucleic acids, preferably SEQ ID NO: 1 and SEQ ID NO: 3 respectively.

In particular, we provide for nucleic acids or polynucleotides which encode any of the *Cricetulus griseus* polypeptides disclosed here. Thus, the terms "cgHSP27 sequence" and "cgHSP70 sequence" should be construed accordingly. Preferably, however, such nucleic acids or polynucleotides comprise any of the sequences set out as SEQ ID NOs: 1 or 3, or a sequence encoding any of the corresponding polypeptides, and a fragment, homologue, variant or derivative of such a nucleic acid. The above terms therefore preferably should be taken to refer to these sequences.

As used here in this document, the terms "polynucleotide", "nucleotide", and nucleic acid are intended to be synonymous with each other. "Polynucleotide" generally refers to any polyribonucleotide or polydeoxribonucleotide, which may be unmodified RNA or DNA or modified RNA or DNA. "Polynucleotides" include, without limitation single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, "polynucleotide" refers to triple-stranded regions comprising RNA or DNA or both RNA and DNA. The term polynucleotide also includes DNAs or RNAs containing one or more modified bases and DNAs or RNAs with backbones modified for stability or for other reasons. "Modified" bases include, for example, tritylated bases and unusual bases such as inosine. A variety of modifications has been made to DNA and RNA; thus, "polynucleotide" embraces chemically, enzymatically or metabolically modified forms of polynucleotides as typically found in nature, as well as the chemical forms of DNA and RNA characteristic of viruses and cells. "Polynucleotide" also embraces relatively short polynucleotides, often referred to as oligonucleotides.

It will be understood by a skilled person that numerous different polynucleotides and nucleic acids can encode the same polypeptide as a result of the degeneracy of the genetic code. In addition, it is to be understood that skilled persons may, using routine techniques, make nucleotide substitutions that do not affect the polypeptide sequence encoded by the polynucleotides described here to reflect the codon usage of any particular host organism in which the polypeptides are to be expressed.

### Variants, Derivatives and Homologues

The polynucleotides described here may comprise DNA or RNA. They may be single-stranded or double-stranded. They may also be polynucleotides which include within them synthetic or modified nucleotides. A number of different types of modification to oligonucleotides are known in the art. These include methylphosphonate and phosphorothioate backbones, addition of acridine or polylysine chains at the 3' and/or 5' ends of the molecule. For the purposes of the present document, it is to be understood that the polynucleotides described herein may be modified by any method available in the art. Such modifications may be carried out in order to enhance the *in vivo* activity or life span of polynucleotides.

Where the polynucleotide is double-stranded, both strands of the duplex, either individually or in combination, are encompassed by the methods and compositions described here. Where the polynucleotide is single-stranded, it is to be understood that the complementary sequence of that polynucleotide is also included.

The terms "variant", "homologue" or "derivative" in relation to a nucleotide sequence include any substitution of, variation of, modification of, replacement of, deletion of or addition of one (or more) nucleotides from or to the sequence. Preferably, the resulting sequence is capable of encoding a polypeptide which has apoptosis mediator activity.

As indicated above, with respect to sequence identity, a "homologue" has preferably at least 5% identity, at least 10% identity, at least 15% identity, at least 20% identity, at least 25% identity, at least 30% identity, at least 35% identity, at least 40% identity, at least 45% identity, at least 50% identity, at least 55% identity, at least 60% identity, at least 65% identity, at least 70% identity, at least 75% identity, at least 80% identity, at least 85% identity, at least 90% identity, or at least 95% identity to the relevant sequence shown in the sequence listings.

More preferably there is at least 95% identity, more preferably at least 96% identity, more preferably at least 97% identity, more preferably at least 98% identity, more preferably at least 99% identity. Nucleotide homology comparisons may be conducted as described above. A preferred sequence comparison program is the GCG Wisconsin Bestfit program described above. The default scoring matrix has a match value of 10 for each identical nucleotide and -9 for each mismatch. The default gap creation penalty is -50 and the default gap extension penalty is -3 for each nucleotide.

In preferred embodiments, a cgHSP27 polynucleotide has at least 98.1% or more sequence identity to a sequence shown as SEQ ID NO: 1. Preferably, the cgHSP27 polynucleotide has 98.2% or more, preferably 98.3% or more, 98.4% or more, 98.5% or more, 98.6% or more, 98.7% or more, 98.8% or more, 98.9% or more, 99.0% or more or 99.1% or more, 99.2% or more, preferably 99.3% or more, 99.4% or more, 99.5% or more, 99.6% or more, 99.7% or more, 99.8% or more, 99.9% or more sequence identity to a sequence shown as SEQ ID NO: 1.

Similarly, in preferred embodiments , a cgHSP70 polynucleotide has at least 98.1% or more sequence identity to a sequence shown as SEQ ID NO: 3. Preferably, the cgHSP27 polynucleotide has 98.2% or more, preferably 98.3% or more, 98.4% or more, 98.5% or more, 98.6% or more, 98.7% or more, 98.8% or more, 98.9% or more, 99.0% or more or 99.1% or more, 99.2% or more, preferably 99.3% or more, 99.4% or more, 99.5% or more, 99.6% or more, 99.7% or more, 99.8% or more, 99.9% or more sequence identity to a sequence shown as SEQ ID NO: 3.

### Hybridisation

We further describe cgHSP27 and cgHSP70 nucleotide sequences that are capable of hybridising selectively to any of the sequences presented herein, or any variant, fragment or derivative thereof, or to the complement of any of the above. Nucleotide sequences are preferably at least 15 nucleotides in length, more preferably at least 20, 30, 40 or 50 nucleotides in length.

The term "hybridisation" as used herein shall include "the process by which a strand of nucleic acid joins with a complementary strand through base pairing" as well as the process of amplification as carried out in polymerase chain reaction technologies.

Polynucleotides capable of selectively hybridising to the nucleotide sequences presented herein, or to their complement, will be generally at least 70%, preferably at least 80 or 90% and more preferably at least 95% or 98% homologous to the corresponding nucleotide sequences presented herein over a region of at least 20, preferably at least 25 or 30, for instance at least 40, 60 or 100 or more contiguous nucleotides.

The term "selectively hybridisable" means that the polynucleotide used as a probe is used under conditions where a target polynucleotide is found to hybridize to the probe at a level significantly above background. The background hybridization may occur because of other polynucleotides present, for example, in the cDNA or genomic DNA library being screened. In this event, background implies a level of signal generated by interaction between the probe and a non-specific DNA member of the library which is less than 10 fold, preferably less than 100 fold as intense as the specific interaction observed with the target DNA. The intensity of interaction may be measured, for example, by radiolabelling the probe, e.g. with ³²P.

Hybridisation conditions are based on the melting temperature (Tm) of the nucleic acid binding complex, as taught in Berger and Kimmel (1987, Guide to Molecular Cloning Techniques, Methods in Enzymology, Vol 152, Academic Press, San Diego CA), and confer a defined "stringency" as explained below.

Maximum stringency typically occurs at about Tm-5°C (5°C below the Tm of the probe); high stringency at about 5°C to 10°C below Tm; intermediate stringency at about 10°C to 20°C below Tm; and low stringency at about 20°C to 25°C below Tm. As will be understood by those of skill in the art, a maximum stringency hybridisation can be used to identify or detect identical polynucleotide sequences while an intermediate (or low) stringency hybridisation can be used to identify or detect similar or related polynucleotide sequences.

In a preferred aspect, we disclose nucleotide sequences that can hybridise to a cgHSP27 and cgHSP70 nucleic acid, or a fragment, homologue, variant or derivative thereof, under stringent conditions (e.g. 65°C and 0.1xSSC {1xSSC = 0.15 M NaCl, 0.015 M Na₃ Citrate pH 7.0}).

Where a polynucleotide is double-stranded, both strands of the duplex, either individually or in combination, are encompassed by the present disclosure. Where the polynucleotide is single-stranded, it is to be understood that the complementary sequence of that polynucleotide is also disclosed and encompassed.

Polynucleotides which are not 100% homologous to the sequences disclosed here but fall within the disclosure can be obtained in a number of ways. Other variants of the sequences described herein may be obtained for example by probing DNA libraries made from a range of individuals, for example individuals from different populations. In addition, other viral/bacterial, or cellular homologues particularly cellular homologues found in mammalian cells (e.g. rat, mouse, bovine and primate cells), may be obtained and such homologues and fragments thereof in general will be capable of selectively hybridising to the sequences shown in the sequence listing herein. Such sequences may be obtained by probing cDNA libraries made from or genomic DNA libraries from other animal species, and probing such libraries with probes comprising all or part of SEQ ID NO: 1 or 3 under conditions of medium to high stringency. Similar considerations apply to obtaining species homologues and allelic variants of cgHSP27 and cgHSP70.

The polynucleotides described here may be used to produce a primer, e.g. a PCR primer, a primer for an alternative amplification reaction, a probe e.g. labelled with a revealing label by conventional means using radioactive or non-radioactive labels, or the polynucleotides may be cloned into vectors. Such primers, probes and other fragments will be at least 15, preferably at least 20, for example at least 25, 30 or 40 nucleotides in length, and are also encompassed by the term polynucleotides as used herein. Preferred fragments are less than 500, 200, 100, 50 or 20 nucleotides in length.

Polynucleotides such as a DNA polynucleotides and probes may be produced recombinantly, synthetically, or by any means available to those of skill in the art. They may also be cloned by standard techniques.

In general, primers will be produced by synthetic means, involving a step wise manufacture of the desired nucleic acid sequence one nucleotide at a time. Techniques for accomplishing this using automated techniques are readily available in the art.

Longer polynucleotides will generally be produced using recombinant means, for example using PCR (polymerase chain reaction) cloning techniques. This will involve making a pair of primers (e.g. of about 15 to 30 nucleotides) flanking a region of the sequence which it is desired to clone, bringing the primers into contact with mRNA or cDNA obtained from an animal or human cell, performing a polymerase chain reaction under conditions which bring about amplification of the desired region, isolating the amplified fragment (e.g. by purifying the reaction mixture on an agarose gel) and recovering the amplified DNA. The primers may be designed to contain suitable restriction enzyme recognition sites so that the amplified DNA can be cloned into a suitable cloning vector

### APOPTOSIS

According to the invention, increase in cell viability of cgHSP27 and/or cgHSP70 modified cells results from a decrease in apoptosis in the modified cell populations.

Accordingly, preferably, apoptosis in a modified cell population is decreased by at least 10%, preferably 25% or more, more preferably 40%, 50%, 75%, 95% or more compared to a control population. In preferred embodiments, the percentage of apoptotic cells in a modified cell population is decreased by such amounts. Methods of assaying apoptosis are known in the art, and are described in detail below and in the Examples. A preferred method of assaying apoptosis is set out in Example 14: Apoptosis Assay.

An alternative assay of apoptosis involves quantitation or measurement of levels any one or more of caspase 2, caspase 3, caspase 8 and caspase 9 in the relevant cells. Thus, preferably, levels of any one or more of these caspases is decreased in a modified cell or population compared to one which has not been so modified, by 10%, 20%, 30%, 50%, 70%, 80%, 90% or more. Preferably, modified cells exhibit a delay in expression of any one or more of caspase 2, caspase 3, caspase 8 and caspase 9 by a period of time preferably at least 1 hour, more preferably at least 6 hours, most preferably at least 12 hours or more, e.g., at least 24 hours, at least 36 hours, at least 48 hours, at least 72 hours, at least 144 hours, at least 288 hours, or more, compared to control cells which are not modified. Caspase levels may be assayed by any means known in the art, including RT-PCR, RNAse protection, SDS-PAGE, immunoassays, etc.

Cell death can occur by either of two distinct mechanisms, necrosis or apoptosis. In addition, certain chemical compounds and cells are said to be cytotoxic to the cell, that is, to cause its death.

"Cytotoxicity" refers to the cell killing property of a chemical compound (such as a food, cosmetic, or pharmaceutical) or a mediator cell (cytotoxic T cell). In contrast to necrosis and apoptosis, the term cytotoxicity need not necessarily indicate a specific cellular death mechanism. For example, cell mediated cytotoxicity (that is, cell death mediated by either cytotoxic T lymphocytes [CTL] or natural killer [NK] cells) combines some aspects of both necrosis and apoptosis.

"Necrosis" (also referred to as "accidental" cell death) refers to the pathological process which occurs when cells are exposed to a serious physical or chemical insult. Necrosis occurs when cells are exposed to extreme variance from physiological conditions (e.g., hypothermia, hypoxia) which may result in damage to the plasma membrane. Under physiological conditions direct damage to the plasma membrane is evoked by agents like complement and lytic viruses. Necrosis begins with an impairment of the cell's ability to maintain homeostasis, leading to an influx of water and extracellular ions. Intracellular organelles, most notably the mitochondria, and the entire cell swell and rupture (cell lysis). Due to the ultimate breakdown of the plasma membrane, the cytoplasmic contents including lysosomal enzymes are released into the extracellular fluid. Therefore, *in vivo,* necrotic cell death is often associated with extensive tissue damage resulting in an intense inflammatory response.

"Apoptosis" ("normal" or "programmed" cell death) refers to the physiological process by which unwanted or useless cells are eliminated during development and other normal biological processes. Apoptosis is a mode of cell death that occurs under normal physiological conditions and the cell is an active participant in its own demise ("cellular suicide"). It is most often found during normal cell turnover and tissue homeostasis, embryogenesis, induction and maintenance of immune tolerance, development of the nervous system and endocrinedependent tissue atrophy. Cells undergoing apoptosis show characteristic morphological and biochemical features. These features include chromatin aggregation, nuclear and cytoplasmic condensation, partition of cytoplasm and nucleus into membrane bound vesicles (apoptotic bodies) which contain ribosomes, morphologically intact mitochondria and nuclear material. *In vivo,* these apoptotic bodies are rapidly recognized and phagocytized by either macrophages or adjacent epithelial cells. Due to this efficient mechanism for the removal of apoptotic cells in vivo no inflammatory response is elicited. *In vitro,* the apoptotic bodies as well as the remaining cell fragments ultimately swell and finally lyse. This terminal phase of in vitro cell death has been termed "secondary necrosis".

Table 1 summarises the various observable differences between necrosis and apoptosis. Preferably, modified cells exhibit a reduction in one or more of these features. Any of these differences, alone or in combination, may be assayed in order to determine whether cell death is occurring by apoptosis or by necrosis.

**Table 1: Differential features and significance of necrosis and apoptosis.**

| | **Necrosis** | **Apoptosis** |
|---|---|---|
| **Morphological features** | Loss of membrane integrity | Membrane blebbing, but no loss of integrity |
| | Begins with swellinof cytoplasm and mitochondria | Aggregation of chromatin at the nuclear membrane |
| | Ends with total cell lysis | Begins with shrinking of cytoplasm and condensation of nucleus |
| | No vesicle formation, complete lysis | Ends with fragmentation of cell into smaller bodies |
| | Disintegration (swelling) of organelles | Formation of membrane bound vesicles (apoptotic bodies) |
| | | Mitochondria become leaky due to pore formation involving proteins of the bcl-2 family. |
| **Biochemical features** | Loss of regulation of ion homeostasis | Tightly regulated process involving activation and enzymatic steps |
| | No energy requirement (passive process, also occurs at 4°C) Random digestion of DNA (smear of DNA after agarose gel electrophoresis) Postlytic DNA fragmentation (= late event of death) | Energy (ATP)-dependent (active process, does not occur at 4°C) |
| | | Non-random mono- and oligonucleosomal length fragmentation of DNA (Ladder pattern after agarose gel electrophoresis) |
| | | Prelytic DNA fragmentation Release of various factors (cytochrome C, AIF) into cytoplasm by mitochondria |
| | | Activation of caspase cascade |
| | | Alterations in membrane asymmetry (i.e., translocation of phosphatidyl-serine |
| | | from the cytoplasmic to the extracellular side of the membrane) |
| **Physiological significance** | Affects groups of contiguous cells Evoked by non-physiological disturbances (complement attack, lytic viruses, hypothermia, hypoxia, ischemica, metabolic poisons) Phagocytosis by macrophages Significant inflammatory response | Affects individual cells |
| | | Induced by physiological stimuli (lack of growth factors, changes in hormonal environment) |
| | | Phagocytosis by adjacent cells or macrophages |
| | | No inflammatory response |

Reference is made to the following documents, which describe apoptosis in detail, as well as various assays for measuring cell death by apoptosis: Schwartzman, R. A. and Cidlowski, J. A. (1993). Endocrine Rev. 14, 133; Vermes, I. and Haanan, C. (1994). Adv. Clin. Chem. 31, 177; Berke, G. (1991). Immunol. Today 12, 396; Krähenbühl, O. and Tschopp, J. (1991). Immunol. Today12, 399; Van Furth, R. and Van Zwet, T. L. (1988). J. Immunol; Methods 108,45. Cohen, J. J. (1993) Apoptosis. Immunol. Today14, 126; Savill, J. S. et al. (1989). J. Clin. Invest. 83, 865; Wyllie, A. H. (1980). Nature 284, 555; Leist, M. et al. (1994) Biochemica No. 3, 18-20; Fraser, A. and Evan, G. (1996) Cell 85, 781-784; Duke, R. C. (1983). Proc. Natl. Acad. Sci. USA 80,6361; Duke, R. C. & Cohen, J. J. (1986). Lymphokine Res. 5, 289; Trauth, B. C. et al. (1994) Eur. J. Cell. Biol. 63, 32,Suppl 40; Matzinger, P. (1991). J. Immunol; Methods 145, 185; Kaeck, M. R. (1993); Anal. Biochem. 208, 393; Prigent, P. et al. (1993). J. Immunol; Methods 160, 139; Huang, P. & Plunkett, W. (1992); Anal. Biochem. 207, 163Bortner, C. D. et al. (1995) Trends Cell Biol. 5, 21; Gold, R. et al. (1994); Lab. Invest. 71, 219.

Apoptosis and cell mediated cytotoxicity are characterized by cleavage of the genomic DNA into discrete fragments prior to membrane disintegration. Accordingly, apoptosis may be assayed by measuring DNA fragmentation, for example, by observing the presence of DNA ladders. DNA fragments may be assayed , for example, as "ladders" (with the 180 bp multiples as "rungs" of the ladder) derived from populations of cells, or by quantification of histone complexed DNA fragments via, for exmaple, ELISA. Such an assay relies on an one-step sandwich immunoassay to detect nucleosomes. The procedure involves pelleting cells by centrifugation and discarding the supernatant (which contains DNA from necrotic cells that leaked through the membrane during incubation). Cells are resuspended and incubated in lysis buffer. After lysis, intact nuclei are pelleted by centrifugation. An aliquot of the supernatant is transferred to a streptavidin-coated well of a microtiter plate, and nucleosomes in the supernatant are bound with two monoclonal antibodies, anti-histone (biotin-labeled) and anti-DNA (peroxidase-conjugated). Antibody-nu-cleosome complexes are bound to the microtiter plate by the streptavidin. The immobilized antibody-histone complexes are washed three times to remove cell components that are not immuno-reactive, and the sample is incubated with peroxidase sub-strate (ABTS ®). The amount of colored product (and thus, of immobilized antibody- histone complexes) is then determined spectrophotometrically.

Several proteases are involved in the early stages of apoptosis. Apoptosis may therefore also be assayed by detecting the presence of, in addition to, or instead of, assaying the activity of, apoptosis-induced proteases such as caspases, e.g., caspase 3. Caspase activation can be analyzed in different ways, for example, by an *in vitro* enzyme assay of, for example, cellular lysates by capturing of the caspase and measuring proteolytic cleavage of a suitable substrate. Furthermore, caspases may be assayed by detection of cleavage of an *in vivo* caspase substrate such as PARP (Poly-ADP-Ribose-Polymer-ase). Cleaved fragments of PARP may be detected with a suitable antibody such as an anti PARP antibody. Protease assays and DNA fragmentation assays are especially suitable for assaying apoptosis in cell populations.

Methods for studying apoptosis in individual cells are also available, such as ISNT and TUNEL enzymatic labeling assays. As noted above, extensive DNA degradation is a characteristic event which often occurs in the early stages of apoptosis. Cleavage of the DNA yields double-stranded, low molecular weight DNA fragments (mono- and oligonucleosomes) as well as single strand breaks ("nicks") in high molecular weight-DNA. In TUNEL, such DNA strand breaks are detected by enzymatic labeling of the free 3'-OH termini with suitable modified nucleotides (such as X-dUTP, X = biotin, DIG or fluorescein). Suitable labeling enzymes include DNA polymerase (nick translation) in ISNT ("*in situ* nick translation") and terminal deoxynucleotidyl transferase (end labeling) in TUNEL ("TdT-mediated X-dUTP nick end labeling"; Huang, P. & Plunkett, W., 1992, Anal. Biochem. 207, 163; Bortner , C. D. et al., 1995, Trends Cell Biol. 5, 21).

Apoptosis may also be assayed by measuring membrane alterations, including: loss of terminal sialic acid residues from the side chains of cell surface glycoproteins, exposing new sugar residues; emergence of surface glycoproteins that may serve as receptors for macrophage-secreted adhesive molecules such as thrombospondin; and loss of asymmetry in cell membrane phospholipids, altering both the hydrophobicity and charge of the membrane surface. In particular, the human anticoagulant annexin V is a 35-36 kilodalton, Ca2+-dependent phospholipid-binding protein that has a high affinity for phosphatidylserine (PS). In normal viable cells, PS is located on the cytoplasmic surface of the cell membrane. However, in apoptotic cells, PS is translocated from the inner to the outer leaflet of the plasma membrane, thus exposing PS to the external cellular environment. Annexin V may therefore be used to detect phos-phatidylserine asymmetrically exposed on the surface of apoptotic cells (Homburg, C. H. E: et al. 1995, Blood 85, 532; Verhoven, B. et al., 1995, J. Exp. Med. 182, 1597). Furthermore, DNA stains such as DAPI, ethidium bromide and propidium iodide, etc may be used for differential staining to distinguish viable and non-viable cells. Profiles of DNA content may also be used; thus, permeabilized apoptotic cells leak low molecular weight DNA, and detection of "sub-G 1 peaks", or "A 0" cells (cells with lower DNA staining than that of G 1 cells) may be detected by, for example, flow cytometry. Morphological changes characteristic of apoptosis may also be detected in this manner.

Detection of apoptosis-related proteins such as ced-3, ced-4, ced-9 (Ellis, H. M. and Horvitz, H. R., 1986, Cell 44, 817-829; Yuan, J. Y. and Horvitz, H. R., 1990, Dev. Biol. 138, 33-41; Hentgartner, M. O., Ellis, R. E. and Horvitz, H. R., 1992, Nature 356, 494-499.), Fas(CD95/Apo-1; Enari et al., 1996, Nature 380, 723-726), Bcl-2 (Baffy, G. et al., 1993, J. Biol. Chem. 268, 6511-6519; Miyashita, T. and Reed, J. C., 1993, Blood 81, 151-157; Oltvai, Z. N., Milliman, C. L. and Korsmeyer, S. J., 1993, Cell 74, 609-619), p53 (Yonish-Rouach, E. et al., 1991, Nature 352, 345-347), etc by the use of antibodies may also be used to assay apoptosis.

### NUCLEOTIDE VECTORS

The polynucleotides disclosed here, specifically cgHSP27 and cgHSP70, can be incorporated into a recombinant replicable vector. The vector may be used to replicate the nucleic acid in a compatible host cell. Thus in a further embodiment, we provide a method of making polynucleotides by introducing a polynucleotide into a replicable vector, introducing the vector into a compatible host cell, and growing the host cell under conditions which bring about replication of the vector. The vector may be recovered from the host cell. Suitable host cells include bacteria such as *E. coli,* yeast, mammalian cell lines and other eukaryotic cell lines, for example insect Sf9 cells.

Preferably, a polynucleotide in a vector is operably linked to a control sequence that is capable of providing for the expression of the coding sequence by the host cell, i.e. the vector is an expression vector. The term "operably linked" means that the components described are in a relationship permitting them to function in their intended manner. A regulatory sequence "operably linked" to a coding sequence is ligated in such a way that expression of the coding sequence is achieved under condition compatible with the control sequences.

The control sequences may be modified, for example by the addition of further transcriptional regulatory elements to make the level of transcription directed by the control sequences more responsive to transcriptional modulators.

The nucleotide vectors, preferably expression vectors, may suitably comprise coding sequences for any protein of interest which it is desired to express, for example, a glycoprotein such as interferon-γ. Alternatively, or in addition, the protein of interest may be expressed from a separate expression vector, which may be constructed in an analogous fashion to the methods described here.

Vectors may be transformed or transfected into a suitable host cell as described below to provide for expression of a protein. This process may comprise culturing a host cell transformed with an expression vector as described above under conditions to provide for expression by the vector of a coding sequence encoding the protein, and optionally recovering the expressed protein.

The vectors may be for example, plasmid or virus vectors provided with an origin of replication, optionally a promoter for the expression of the said polynucleotide and optionally a regulator of the promoter. The vectors may contain one or more selectable marker genes, for example an ampicillin resistance gene in the case of a bacterial plasmid or a neomycin resistance gene for a mammalian vector. Vectors may be used, for example, to transfect or transform a host cell.

Control sequences operably linked to sequences encoding the protein include promoters/enhancers and other expression regulation signals. These control sequences may be selected to be compatible with the host cell for which the expression vector is designed to be used in. The term "promoter" is well-known in the art and encompasses nucleic acid regions ranging in size and complexity from minimal promoters to promoters including upstream elements and enhancers.

The promoter is typically selected from promoters which are functional in mammalian cells, although prokaryotic promoters and promoters functional in other eukaryotic cells may be used. The promoter is typically derived from promoter sequences of viral or eukaryotic genes. For example, it may be a promoter derived from the genome of a cell in which expression is to occur. With respect to eukaryotic promoters, they may be promoters that function in a ubiquitous manner (such as promoters of α-actin, β-actin, tubulin) or, alternatively, a tissue-specific manner (such as promoters of the genes for pyruvate kinase). They may also be promoters that respond to specific stimuli, for example promoters that bind steroid hormone receptors. Viral promoters may also be used, for example the Moloney murine leukaemia virus long terminal repeat (MMLV LTR) promoter, the Rous sarcoma virus (RSV) LTR promoter or the human cytomegalovirus (CMV) IE promoter.

It may also be advantageous for the promoters to be inducible so that the levels of expression of the heterologous gene can be regulated during the life-time of the cell. Inducible means that the levels of expression obtained using the promoter can be regulated.

In addition, any of these promoters may be modified by the addition of further regulatory sequences, for example enhancer sequences. Chimeric promoters may also be used comprising sequence elements from two or more different promoters described above.

### EXPRESSION OF CGHSP27 AND CGHSP70 POLYPEPTIDES

In order to express a biologically active cgHSP27 and/or cgHSP70 polypeptide, a *Cricetulus griseus* cgHSP27 and/or cgHSP70 polynucleotide sequence is brought into association with a regulatory sequence so as to enable the regulatory sequence to direct expression of said polynucleotide. Expression of the polypeptide under control of the regulatory sequence is then allowed to happen. Optionally, the polypeptide so produced may be purified.

Preferably, the regulatory sequence is one with which the cgHSP27 and cgHSP70 polynucleotide sequence is not naturally associated.

We therefore describe a method of producing polypeptide comprising providing a cell, preferably a *Cricetulus griseus* cell, in which a *Cricetulus griseus* cgHSP27 and cgHSP70 polynucleotide sequence has been brought into association with a regulatory sequence so as to enable the regulatory sequence to direct expression of said polynucleotide, and culturing the cell under conditions which enable expression of the polypeptide, and optionally purifying the polypeptide.

We further describe a method of producing a polypeptide comprising: (a) providing an expression sequence produced by bringing a *Cricetulus griseus* cgHSP27 and cgHSP70 polynucleotide sequence into association with a regulatory sequence so as to enable the regulatory sequence to direct expression of said polynucleotide; (b) allowing expression of the polypeptide from the expression sequence under control of the regulatory sequence, and (c) optionally purifying the polypeptide.

In particular, the nucleotide sequences encoding the respective nucleic acid or homologues, variants, or derivatives thereof may be inserted into appropriate expression vector, i.e., a vector which contains the necessary elements for the transcription and translation of the inserted coding sequence.

We also provide for a polypeptide produced by any of the above methods.

Methods of enabling expression of cgHSP27 and cgHSP70 polypeptides are set out below. It will be appreciated that these methods may be suitable for use in embodiments of the methods and compositions described here in which up-regulation of a polypeptide is desired, e.g., up-regulation of cgHSP27 and/or cgHSP70 in order to achieve enhanced cell viability.

One method by which to provide expressed polypeptides is by means of an expression vector, i.e., a vector (e.g., a plasmid) which contains a regulatable promoter, optionally with other regulatory sequences such as enhancers, which is operably linked to a sequence encoding a polypeptide of interest which has been cloned into the expression vector.

Methods which are well known to those skilled in the art may be used to construct expression vectors containing sequences encoding cgHSP27 and cgHSP70 and appropriate transcriptional and translational control elements. These methods include *in vitro* recombinant DNA techniques, synthetic techniques, and in vivo genetic recombination. Such techniques are described in Sambrook, J. et al. (1989; Molecular Cloning, A Laboratory Manual, ch. 4, 8, and 16-17, Cold Spring Harbor Press, Plainview, N.Y.) and Ausubel, F. M. et al. (1995 and periodic supplements; Current Protocols in Molecular Biology, ch. 9, 13, and 16, John Wiley & Sons, New York, N.Y.).

A variety of expression vector/host systems may be utilized to contain and express sequences encoding cgHSP27 and cgHSP70. These include, but are not limited to, microorganisms such as bacteria transformed with recombinant bacteriophage, plasmid, or cosmid DNA expression vectors; yeast transformed with yeast expression vectors; insect cell systems infected with virus expression vectors (e.g., baculovirus); plant cell systems transformed with virus expression vectors (e.g., cauliflower mosaic virus (CaMV) or tobacco mosaic virus (TMV)) or with bacterial expression vectors (e.g., Ti or pBR322 plasmids); or animal cell systems. Any suitable host cell may be employed.

The "control elements" or "regulatory sequences" are those non-translated regions of the vector (i.e., enhancers, promoters, and 5' and 3' untranslated regions) which interact with host cellular proteins to carry out transcription and translation. Such elements may vary in their strength and specificity. Depending on the vector system and host utilized, any number of suitable transcription and translation elements, including constitutive and inducible promoters, may be used. For example, when cloning in bacterial systems, inducible promoters such as the hybrid lacZ promoter of the BLUESCRIPT phagemid (Stratagene, La Jolla, Calif.) or PSPORT1 plasmid (GIBCO/BRL), and the like, may be used. The baculovirus polyhedrin promoter may be used in insect cells. Promoters or enhancers derived from the genomes of plant cells (e.g., heat shock, RUBISCO, and storage protein genes) or from plant viruses (e.g., viral promoters or leader sequences) may be cloned into the vector.

In mammalian cell systems, promoters from mammalian genes or from mammalian viruses are preferable. If it is necessary to generate a cell line that contains multiple copies of the sequence encoding cgHSP27 and cgHSP70, vectors based on SV40 or EBV may be used with an appropriate selectable marker.

In bacterial systems, a number of expression vectors may be selected depending upon the use intended for cgHSP27 and cgHSP70. For example, when large quantities of cgHSP27 and cgHSP70 are needed for the induction of antibodies, vectors which direct high level expression of fusion proteins that are readily purified may be used. Such vectors include, but are not limited to, multifunctional E. coli cloning and expression vectors such as BLUESCRIPT (Stratagene), in which the sequence encoding cgHSP27 and cgHSP70 may be ligated into the vector in frame with sequences for the amino-terminal Met and the subsequent 7 residues of β-galactosidase so that a hybrid protein is produced, pIN vectors (Van Heeke, G. and S. M. Schuster (1989) J. Biol. Chem. 264:5503-5509), and the like. pGEX vectors (Promega, Madison, Wis.) may also be used to express foreign polypeptides as fusion proteins with glutathione S-transferase (GST). In general, such fusion proteins are soluble and can easily be purified from lysed cells by adsorption to glutathione-agarose beads followed by elution in the presence of free glutathione. Proteins made in such systems may be designed to include heparin, thrombin, or factor XA protease cleavage sites so that the cloned polypeptide of interest can be released from the GST moiety at will.

In the yeast *Saccharomyces cerevisiae*, a number of vectors containing constitutive or inducible promoters, such as alpha factor, alcohol oxidase, and PGH, may be used. For reviews, see Ausubel (supra) and Grant et al. (1987; Methods Enzymol. 153:516-544).

In cases where plant expression vectors are used, the expression of sequences encoding cgHSP27 and cgHSP70 may be driven by any of a number of promoters. For example, viral promoters such as the 35S and 19S promoters of CaMV may be used alone or in combination with the omega leader sequence from TMV. (Takamatsu, N. (1987) EMBO J. 6:307-311.) Alternatively, plant promoters such as the small subunit of RUBISCO or heat shock promoters may be used. (Coruzzi, G. et al. (1984) EMBO J. 3:1671-1680; Broglie, R. et al. (1984) Science 224:838-843; and Winter, J. et al. (1991) Results Probl. Cell Differ. 17:85-105.) These constructs can be introduced into plant cells by direct DNA transformation or pathogen-mediated transfection. Such techniques are described in a number of generally available reviews. (See, for example, Hobbs, S. or Murry, L. E. in McGraw Hill Yearbook of Science and Technology (1992) McGraw Hill, New York, N.Y.; pp. 191-196.).

An insect system may also be used to express cgHSP27 and/or cgHSP70. For example, in one such system, *Autographa californica* nuclear polyhedrosis virus (AcNPV) is used as a vector to express foreign genes in *Spodoptera frugiperda* cells or in *Trichoplusia* larvae. The sequences encoding cgHSP27 and/or cgHSP70 may be cloned into a non-essential region of the virus, such as the polyhedrin gene, and placed under control of the polyhedrin promoter. Successful insertion of cgHSP27 and/or cgHSP70 will render the polyhedrin gene inactive and produce recombinant virus lacking coat protein. The recombinant viruses may then be used to infect, for example, *S. frugiperda* cells or *Trichoplusia* larvae in which cgHSP27 and/or cgHSP70 may be expressed. (Engelhard, E. K. et al. (1994) Proc. Nat. Acad. Sci. 91:3224-3227.)

In mammalian host cells, a number of viral-based expression systems may be utilized. In cases where an adenovirus is used as an expression vector, sequences encoding cgHSP27 and/or cgHSP70 may be ligated into an adenovirus transcription/translation complex consisting of the late promoter and tripartite leader sequence. Insertion in a non-essential E1 or E3 region of the viral genome may be used to obtain a viable virus which is capable of expressing cgHSP27 and/or cgHSP70 in infected host cells. (Logan, J. and T. Shenk (1984) Proc. Natl. Acad. Sci. 81:3655-3659.) In addition, transcription enhancers, such as the Rous sarcoma virus (RSV) enhancer, may be used to increase expression in mammalian host cells.

Thus, for example, the cgHSP27 and/or cgHSP70 proteins are expressed in either human embryonic kidney 293 (HEK293) cells or adherent dhfr CHO cells. To maximize receptor expression, typically all 5' and 3' untranslated regions (UTRs) are removed from the receptor cDNA prior to insertion into a pCDN or pCDNA3 vector. The cells are transfected with individual receptor cDNAs by lipofectin and selected in the presence of 400 mg/ml G418. After 3 weeks of selection, individual clones are picked and expanded for further analysis. HEK293 or CHO cells transfected with the vector alone serve as negative controls. To isolate cell lines stably expressing the individual receptors, about 24 clones are typically selected and analyzed by Northern blot analysis. Receptor mRNAs are generally detectable in about 50% of the G418-resistant clones analyzed.

Human artificial chromosomes (HACs) may also be employed to deliver larger fragments of DNA than can be contained and expressed in a plasmid. HACs of about 6 kb to 10 Mb are constructed and delivered via conventional delivery methods (liposomes, polycationic amino polymers, or vesicles) for therapeutic purposes.

Specific initiation signals may also be used to achieve more efficient translation of sequences encoding cgHSP27 and/or cgHSP70. Such signals include the ATG initiation codon and adjacent sequences. In cases where sequences encoding cgHSP27 and/or cgHSP70 and its initiation codon and upstream sequences are inserted into the appropriate expression vector, no additional transcriptional or translational control signals may be needed. However, in cases where only coding sequence, or a fragment thereof, is inserted, exogenous translational control signals including the ATG initiation codon should be provided. Furthermore, the initiation codon should be in the correct reading frame to ensure translation of the entire insert. Exogenous translational elements and initiation codons may be of various origins, both natural and synthetic. The efficiency of expression may be enhanced by the inclusion of enhancers appropriate for the particular cell system used, such as those described in the literature. (Scharf, D. et al. (1994) Results Probl. Cell Differ. 20:125-162.)

In addition, a host cell strain may be chosen for its ability to modulate expression of the inserted sequences or to process the expressed protein in the desired fashion. Such modifications of the polypeptide include, but are not limited to, acetylation, carboxylation, glycosylation, phosphorylation, lipidation, and acylation. Post-translational processing which cleaves a "prepro" form of the protein may also be used to facilitate correct insertion, folding, and/or function. Different host cells which have specific cellular machinery and characteristic mechanisms for post-translational activities (e.g., CHO, HeLa, MDCK, HEK293, and WI38), are available from the American Type Culture Collection (ATCC, Bethesda, Md.) and may be chosen to ensure the correct modification and processing of the foreign protein.

For long term, high yield production of recombinant proteins, stable expression is preferred. For example, cell lines capable of stably expressing cgHSP27 and/or cgHSP70 can be transformed using expression vectors which may contain viral origins of replication and/or endogenous expression elements and a selectable marker gene on the same or on a separate vector. Following the introduction of the vector, cells may be allowed to grow for about 1 to 2 days in enriched media before being switched to selective media. The purpose of the selectable marker is to confer resistance to selection, and its presence allows growth and recovery of cells which successfully express the introduced sequences. Resistant clones of stably transformed cells may be proliferated using tissue culture techniques appropriate to the cell type.

Any number of selection systems may be used to recover transformed cell lines. These include, but are not limited to, the herpes simplex virus thymidine kinase genes (Wigler, M. et al. (1977) Cell 11:223-32) and adenine phosphoribosyltransferase genes (Lowy, 1. et al. (1980) Cell 22:817-23), which can be employed in tk- or apr cells, respectively. Also, antimetabolite, antibiotic, or herbicide resistance can be used as the basis for selection. For example, dhfr confers resistance to methotrexate (Wigler, M. et al. (1980) Proc. Natl. Acad. Sci. 77:3567-70); npt confers resistance to the aminoglycosides neomycin and G-418 (Colbere-Garapin, F. et al (1981) J. Mol. Biol. 150:1-14); and als or pat confer resistance to chlorsulfuron and phosphinotricin acetyltransferase, respectively (Murry, supra). Additional selectable genes have been described, for example, trpB, which allows cells to utilize indole in place of tryptophan, or hisD, which allows cells to utilize histinol in place of histidine. (Hartman, S. C. and R. C. Mulligan (1988) Proc. Natl. Acad. Sci. 85:8047-51.) Recently, the use of visible markers has gained popularity with such markers as anthocyanins, β-glucuronidase and its substrate GUS, and luciferase and its substrate luciferin. These markers can be used not only to identify transformants, but also to quantify the amount of transient or stable protein expression attributable to a specific vector system. (Rhodes, C. A. et al. (1995) Methods Mol. Biol. 55:121-131.)

Although the presence/absence of marker gene expression suggests that the gene of interest is also present, the presence and expression of the gene may need to be confirmed. For example, if the sequence encoding cgHSP27 and/or cgHSP70 is inserted within a marker gene sequence, transformed cells containing sequences encoding cgHSP27 and/or cgHSP70 can be identified by the absence of marker gene function. Alternatively, a marker gene can be placed in tandem with a sequence encoding cgHSP27 and/or cgHSP70 under the control of a single promoter. Expression of the marker gene in response to induction or selection usually indicates expression of the tandem gene as well.

Alternatively, host cells which contain the nucleic acid sequence encoding cgHSP27 and/or cgHSP70 and express cgHSP27 and/or cgHSP70 may be identified by a variety of procedures known to those of skill in the art. These procedures include, but are not limited to, DNA--DNA or DNA-RNA hybridizations and protein bioassay or immunoassay techniques which include membrane, solution, or chip based technologies for the detection and/or quantification of nucleic acid or protein sequences.

The presence of polynucleotide sequences encoding cgHSP27 and/or cgHSP70 can be detected by DNA--DNA or DNA-RNA hybridization or amplification using probes or fragments or fragments of polynucleotides encoding cgHSP27 and/or cgHSP70. Nucleic acid amplification based assays involve the use of oligonucleotides or oligomers based on the sequences encoding cgHSP27 and/or cgHSP70 to detect transformants containing DNA or RNA encoding cgHSP27 and/or cgHSP70.

A variety of protocols for detecting and measuring the expression of cgHSP27 and/or cgHSP70, using either polyclonal or monoclonal antibodies specific for the protein, are known in the art. Examples of such techniques include enzyme-linked immunosorbent assays (ELISAs), radioimmunoassays (RIAs), and fluorescence activated cell sorting (FACS). A two-site, monoclonal-based immunoassay utilizing monoclonal antibodies reactive to two non-interfering epitopes on cgHSP27 and/or cgHSP70 is preferred, but a competitive binding assay may be employed. These and other assays are well described in the art, for example, in Hampton, R. et al. (1990; Serological Methods, a Laboratory Manual, Section IV, APS Press, St Paul, Minn.) and in Maddox, D. E. et al. (1983; J. Exp. Med. 158:1211-1216).

A wide variety of labels and conjugation techniques are known by those skilled in the art and may be used in various nucleic acid and amino acid assays. Means for producing labeled hybridization or PCR probes for detecting sequences related to polynucleotides encoding cgHSP27 and/or cgHSP70 include oligolabeling, nick translation, end-labeling, or PCR amplification using a labeled nucleotide. Alternatively, the sequences encoding cgHSP27 and/or cgHSP70, or any fragments thereof, may be cloned into a vector for the production of an mRNA probe. Such vectors are known in the art, are commercially available, and may be used to synthesize RNA probes in vitro by addition of an appropriate RNA polymerase such as T7, T3, or SP6 and labeled nucleotides. These procedures may be conducted using a variety of commercially available kits, such as those provided by Pharmacia & Upjohn (Kalamazoo, Mich.), Promega (Madison, Wis.), and U.S. Biochemical Corp. (Cleveland, Ohio). Suitable reporter molecules or labels which may be used for ease of detection include radionuclides, enzymes, fluorescent, chemiluminescent, or chromogenic agents, as well as substrates, cofactors, inhibitors, magnetic particles, and the like.

Host cells transformed with nucleotide sequences encoding cgHSP27 and/or cgHSP70 may be cultured under conditions suitable for the expression and recovery of the protein from cell culture. The protein produced by a transformed cell may be located in the cell membrane, secreted or contained intracellularly depending on the sequence and/or the vector used. As will be understood by those of skill in the art, expression vectors containing polynucleotides which encode cgHSP27 and/or cgHSP70 may be designed to contain signal sequences which direct secretion of cgHSP27 and/or cgHSP70 through a prokaryotic or eukaryotic cell membrane. Other constructions may be used to join sequences encoding cgHSP27 and/or cgHSP70 to nucleotide sequences encoding a polypeptide domain which will facilitate purification of soluble proteins. Such purification facilitating domains include, but are not limited to, metal chelating peptides such as histidine-tryptophan modules that allow purification on immobilized metals, protein A domains that allow purification on immobilized immunoglobulin, and the domain utilized in the FLAGS extension/affinity purification system (Immunex Corp., Seattle, Wash.). The inclusion of cleavable linker sequences, such as those specific for Factor XA or enterokinase (Invitrogen, San Diego, Calif.), between the purification domain and the cgHSP27 and/or cgHSP70 encoding sequence may be used to facilitate purification. One such expression vector provides for expression of a fusion protein containing cgHSP27 and/or cgHSP70 and a nucleic acid encoding 6 histidine residues preceding a thioredoxin or an enterokinase cleavage site. The histidine residues facilitate purification on immobilized metal ion affinity chromatography (IMIAC; described in Porath, J. et al. (1992) Prot. Exp. Purif. 3: 263-281), while the enterokinase cleavage site provides a means for purifying cgHSP27 and/or cgHSP70 from the fusion protein. A discussion of vectors which contain fusion proteins is provided in Kroll, D. J. et al. (1993; DNA Cell Biol. 12:441-453).

Fragments of cgHSP27 and/or cgHSP70, as well as whole length polypeptides, may be produced not only by recombinant production, but also by direct peptide synthesis using solid-phase techniques. (Merrifield J. (1963) J. Am. Chem. Soc. 85:2149-2154.) Protein synthesis may be performed by manual techniques or by automation. Automated synthesis may be achieved, for example, using the Applied Biosystems 431A peptide synthesizer (Perkin Elmer). Various fragments of cgHSP27 and/or cgHSP70 may be synthesized separately and then combined to produce the full length molecule.

Other methods of expression are also known, for example, a method known as "gene activation" may be employed to modulate activity or expression of cgHSP27 and/or cgHSP70. This method is described in detail in US Patent Number 5,641,670, hereby incorporated by reference. In essence, the gene activation method is based upon the recognition that the regulation or activity of endogenous genes of interest in a cell can be altered by inserting into the cell genome, at a preselected site, through homologous recombination, a suitable DNA construct comprising: (a) a targeting sequence; (b) a regulatory sequence; (c) an exon and (d) an unpaired splice-donor site, wherein the targeting sequence directs the integration of elements (a)-(d) such that the elements (b)-(d) are operatively linked to the endogenous gene. The DNA construct may alternatively comprise: (a) a targeting sequence, (b) a regulatory sequence, (c) an exon, (d) a splice-donor site, (e) an intron, and (f) a splice-acceptor site, wherein the targeting sequence directs the integration of elements (a)-(f) such that the elements of (b)-(f) are operatively linked to the first exon of the endogenous gene.

The targeting sequences used are selected with reference to the site into which the DNA is to be inserted. In both arrangements the targeting event is used to create a new transcription unit, which is a fusion product of sequences introduced by the targeting DNA constructs and the endogenous cellular gene. For example, the formation of the new transcription unit allows transcriptionally silent genes (genes not expressed in a cell prior to transfection) to be activated in host cells by introducing into the host cell's genome a DNA construct as described. The expression of an endogenous gene such as cgHSP27 and/or cgHSP70 which is expressed in a cell as obtained can be altered in that it is increased, reduced, including eliminated, or the pattern of regulation or induction may be changed through use of the gene activation method.

### ANTIBODIES

Specific antagonists of cgHSP27 and/or cgHSP70, which may be used to regulate the activity of these proteins and may include antibodies against the protein(s). In particular, we disclose antibodies capable of binding to cgHSP27 and/or cgHSP70, and preferably capable of inhibiting any biological activity thereof..

We therefore provide in particular for anti- cgHSP27 and/or cgHSP70 antibodies, as well as methods of producing them.

Antibodies, as used herein, refers to complete antibodies or antibody fragments capable of binding to a selected target, and including Fv, ScFv, Fab' and F(ab')₂, monoclonal and polyclonal antibodies, engineered antibodies including chimeric, CDR-grafted and humanised antibodies, and artificially selected antibodies produced using phage display or alternative techniques. Small fragments, such as Fv and ScFv, possess advantageous properties for diagnostic and therapeutic applications on account of their small size and consequent superior tissue distribution.

The anti-cgHSP27 and/or cgHSP70 antibodies described here may be used for the detection of the relevant protein, for example, within the context of a cell. Accordingly, they may be altered antibodies comprising an effector protein such as a label. Especially preferred are labels which allow the imaging of the distribution of the antibody *in vivo* or *in vitro.* Such labels may be radioactive labels or radioopaque labels, such as metal particles, which are readily visualisable within an embryo or a cell mass. Moreover, they may be fluorescent labels or other labels which are visualisable on tissue samples.

Recombinant DNA technology may be used to improve the antibodies as described here. Thus, chimeric antibodies may be constructed in order to decrease the immunogenicity thereof in diagnostic or therapeutic applications. Moreover, immunogenicity may be minimised by humanising the antibodies by CDR grafting [see European Patent Application 0 239 400 (Winter)] and, optionally, framework modification [EP 0 239 400].

Anti-cgHSP27 and/or cgHSP70 antibodies may be obtained from animal serum, or, in the case of monoclonal antibodies or fragments thereof, produced in cell culture. Recombinant DNA technology may be used to produce the antibodies according to established procedure, in bacterial or preferably mammalian cell culture. The selected cell culture system preferably secretes the antibody product.

Therefore, we disclose a process for the production of an antibody comprising culturing a host, e.g. E. coli or a mammalian cell, which has been transformed with a hybrid vector comprising an expression cassette comprising a promoter operably linked to a first DNA sequence encoding a signal peptide linked in the proper reading frame to a second DNA sequence encoding said antibody protein, and isolating said protein.

Multiplication of hybridoma cells or mammalian host cells in vitro is carried out in suitable culture media, which are the customary standard culture media, for example Dulbecco's Modified Eagle Medium (DMEM) or RPMI 1640 medium, optionally replenished by a mammalian serum, e.g. foetal calf serum, or trace elements and growth sustaining supplements, e.g. feeder cells such as normal mouse peritoneal exudate cells, spleen cells, bone marrow macrophages, 2-aminoethanol, insulin, transferrin, low density lipoprotein, oleic acid, or the like. Multiplication of host cells which are bacterial cells or yeast cells is likewise carried out in suitable culture media known in the art, for example for bacteria in medium LB, NZCYM, NZYM, NZM, Terrific Broth, SOB, SOC, 2 x YT, or M9 Minimal Medium, and for yeast in medium YPD, YEPD, Minimal Medium, or Complete Minimal Dropout Medium.

In vitro production provides relatively pure antibody preparations and allows scale-up to give large amounts of the desired antibodies. Techniques for bacterial cell, yeast or mammalian cell cultivation are known in the art and include homogeneous suspension culture, e.g. in an airlift reactor or in a continuous stirrer reactor, or immobilised or entrapped cell culture, e.g. in hollow fibres, microcapsules, on agarose microbeads or ceramic cartridges.

Large quantities of the desired antibodies can also be obtained by multiplying mammalian cells in vivo. For this purpose, hybridoma cells producing the desired antibodies are injected into histocompatible mammals to cause growth of antibody-producing tumours. Optionally, the animals are primed with a hydrocarbon, especially mineral oils such as pristane (tetramethyl-pentadecane), prior to the injection. After one to three weeks, the antibodies are isolated from the body fluids of those mammals. For example, hybridoma cells obtained by fusion of suitable myeloma cells with antibody-producing spleen cells from Balb/c mice, or transfected cells derived from hybridoma cell line Sp2/0 that produce the desired antibodies are injected intraperitoneally into Balb/c mice optionally pre-treated with pristane, and, after one to two weeks, ascitic fluid is taken from the animals.

The foregoing, and other, techniques are discussed in, for example, Kohler and Milstein, (1975) Nature 256:495-497; US 4,376,110; Harlow and Lane, Antibodies: a Laboratory Manual, (1988) Cold Spring Harbor, incorporated herein by reference. Techniques for the preparation of recombinant antibody molecules is described in the above references and also in, for example, EP 0623679; EP 0368684 and EP 0436597, which are incorporated herein by reference.

The cell culture supernatants are screened for the desired antibodies, for example by immunoblotting, by an enzyme immunoassay, e.g. a sandwich assay or a dot-assay, or a radioimmmoassay.

For isolation of the antibodies, the immunoglobulins in the culture supernatants or in the ascitic fluid may be concentrated, e.g. by precipitation with ammonium sulphate, dialysis against hygroscopic material such as polyethylene glycol, filtration through selective membranes, or the like. If necessary and/or desired, the antibodies are purified by the customary chromatography methods, for example gel filtration, ion-exchange chromatography, chromatography over DEAE-cellulose and/or (immuno-) affinity chromatography, e.g. affinity chromatography with cgHSP27 and/or cgHSP70, or fragments thereof, or with Protein-A.

Hybridoma cells secreting the monoclonal antibodies are also provided. Preferred hybridoma cells are genetically stable, secrete monoclonal antibodies of the desired specificity and can be activated from deep-frozen cultures by thawing and recloning.

Also included is a process for the preparation of a hybridoma cell line secreting monoclonal antibodies directed to cgHSP27 and/or cgHSP70, characterised in that a suitable mammal, for example a Balb/c mouse, is immunised with a one or more cgHSP27 and/or cgHSP70 polypeptides, or antigenic fragments thereof; antibody-producing cells of the immunised mammal are fused with cells of a suitable myeloma cell line, the hybrid cells obtained in the fusion are cloned, and cell clones secreting the desired antibodies are selected. For example spleen cells of Balb/c mice immunised with cgHSP27 and/or cgHSP70 are fused with cells of the myeloma cell line PAI or the myeloma cell line Sp2/0-Ag14, the obtained hybrid cells are screened for secretion of the desired antibodies, and positive hybridoma cells are cloned.

Preferred is a process for the preparation of a hybridoma cell line, characterised in that Balb/c mice are immunised by injecting subcutaneously and/or intraperitoneally between 10 and 10⁷ and 10⁸ cells expressing cgHSP27 and/or cgHSP70 and a suitable adjuvant several times, e.g. four to six times, over several months, e.g. between two and four months, and spleen cells from the immunised mice are taken two to four days after the last injection and fused with cells of the myeloma cell line PAI in the presence of a fusion promoter, preferably polyethylene glycol. Preferably the myeloma cells are fused with a three- to twentyfold excess of spleen cells from the immunised mice in a solution containing about 30 % to about 50 % polyethylene glycol of a molecular weight around 4000. After the fusion the cells are expanded in suitable culture media as described hereinbefore, supplemented with a selection medium, for example HAT medium, at regular intervals in order to prevent normal myeloma cells from overgrowing the desired hybridoma cells.

Recombinant DNAs comprising an insert coding for a heavy chain variable domain and/or for a light chain variable domain of antibodies directed to cgHSP27 and/or cgHSP70 as described hereinbefore are also disclosed. By definition such DNAs comprise coding single stranded DNAs, double stranded DNAs consisting of said coding DNAs and of complementary DNAs thereto, or these complementary (single stranded) DNAs themselves.

Furthermore, DNA encoding a heavy chain variable domain and/or for a light chain variable domain of antibodies directed to cgHSP27 and/or cgHSP70 can be enzymatically or chemically synthesised DNA having the authentic DNA sequence coding for a heavy chain variable domain and/or for the light chain variable domain, or a mutant thereof. A mutant of the authentic DNA is a DNA encoding a heavy chain variable domain and/or a light chain variable domain of the above-mentioned antibodies in which one or more amino acids are deleted or exchanged with one or more other amino acids. Preferably said modification(s) are outside the CDRs of the heavy chain variable domain and/or of the light chain variable domain of the antibody. Such a mutant DNA is also intended to be a silent mutant wherein one or more nucleotides are replaced by other nucleotides with the new codons coding for the same amino acid(s). Such a mutant sequence is also a degenerated sequence. Degenerated sequences are degenerated within the meaning of the genetic code in that an unlimited number of nucleotides are replaced by other nucleotides without resulting in a change of the amino acid sequence originally encoded. Such degenerated sequences may be useful due to their different restriction sites and/or frequency of particular codons which are preferred by the specific host, particularly *E. coli,* to obtain an optimal expression of the heavy chain murine variable domain and/or a light chain murine variable domain.

The term mutant is intended to include a DNA mutant obtained by in vitro mutagenesis of the authentic DNA according to methods known in the art.

For the assembly of complete tetrameric immunoglobulin molecules and the expression of chimeric antibodies, the recombinant DNA inserts coding for heavy and light chain variable domains are fused with the corresponding DNAs coding for heavy and light chain constant domains, then transferred into appropriate host cells, for example after incorporation into hybrid vectors.

Also disclosed are recombinant DNAs comprising an insert coding for a heavy chain murine variable domain of an antibody directed to cgHSP27 and/or cgHSP70 fused to a human constant domain g, for example γ1, γ2, γ3 or γ4, preferably γ1 or γ4. Likewise recombinant DNAs comprising an insert coding for a light chain murine variable domain of an antibody directed to cgHSP27 and/or cgHSP70 fused to a human constant domain κ or λ, preferably κ are also disclosed.

In another embodiment, we disclose recombinant DNAs coding for a recombinant polypeptide wherein the heavy chain variable domain and the light chain variable domain are linked by way of a spacer group, optionally comprising a signal sequence facilitating the processing of the antibody in the host cell and/or a DNA coding for a peptide facilitating the purification of the antibody and/or a cleavage site and/or a peptide spacer and/or an effector molecule.

The DNA coding for an effector molecule is intended to be a DNA coding for the effector molecules useful in diagnostic or therapeutic applications. Thus, effector molecules which are toxins or enzymes, especially enzymes capable of catalysing the activation of prodrugs, are particularly indicated. The DNA encoding such an effector molecule has the sequence of a naturally occurring enzyme or toxin encoding DNA, or a mutant thereof, and can be prepared by methods well known in the art.

### EXAMPLES

### Example 1. Cloning of CHO Heat Shock Proteins (HSP) - Materials and Methods

Chinese hamster ovary, CHO-K1, cells (ATCC CCL-61) grown in shakeflask are subjected to heat shock treatment on day 3 of the culture at 42°C for 15 minutes in a water bath. The cells are then returned to the normal growth conditions at 37°C with 8% CO₂ for 90 minutes for cells to initiate heat shock protein expression. 3 X 10⁷ cells are then centrifuged at 1000 rpm for 10 minutes and cell pellets collected for RNA extraction.

Total RNA is extracted from the cell pellets collected using Trizol® reagent according to manufacturer's instructions (Invitrogen). 1^{st}-strand cDNA is synthesized by reverse-transcription of total RNA (30 µg) using ImProm-II reverse transcriptase (Promega) and anchored oligo-dT28 primers (Proligo). The resulting cDNA pool is used directly for PCR amplification.

Primers are designed as shown in Table E1 to amplify the entire coding region of each gene.

**Table E1. Primers for amplifying HSP27 and HSP70 sequences from CHO cells.**

| **Gene** | **Sequences used for Primer Design** | | **Forward Primer** | **Reverse Primer** |
|---|---|---|---|---|
| | **Homologues** | **Accession** | **(5' - 3')** | **(5' - 3')** |
| | Human HSP27 | NM_001540 | CGATGACCGAGCGCCG CGT | TCTACTTGGCTCCAGAC TGTTCAG |
| HSP27 | Mouse HSP27 | NM_013560 | | |
| | Rat HSP27 | NM_031970 | | |
| | Human HSP70 | NM_005345 | CAGCCCGGACGCAGC CTTCCAG | AGCCAGCAGAGGCCT CTAATCC |
| HSP70 | Mouse HSP70 | NM_010479 | | |
| | Rat HSP70 | NM_031971 | | |

The PCR amplifications are performed using Taq DNA polymerase (Fermentas) and the PCR product separated on agarose gel. The amplified DNA fragments are then purified from the agarose gels and subcloned into pCR-TOPO2.1 vector using the TOPO-TA cloning kit according to manufacturer's instructions (Invitrogen) to obtain TOPO-CHO-HSP27 and TOPO-CHO-HSP70. The inserts are sequenced using Big Dye Version 3.1 Terminator kit (Applied Biosystems) on an automated DNA sequencer, ABI PRISM 3100 Genetic Analyzer (Applied Biosystems).

### Example 2. Cloning of Novel CHO Heat Shock Proteins, HSP27 and HSP70

Cloning and characterization of several members of the Chinese Hamster (*Cricetulus griseus*) heat shock protein 70 kDa family, eg. HSC70 (Genbank Accession: M34561) and mitochondrial HSP70 (Genbank Accession: U92313), have been detailed in literature (Suhail *et al.* 1990, Singh *et al.* 1997). However, the gene sequences for the inducible forms of the heat shock proteins 27kDa and 70kDa (HSP27 and HSP70) have not previously been deposited in Genbank.

Primers are designed against HSP27 and HSP70 as described. The genes are then cloned from CHO-K1 cells using mRNA extracted from cells exposed to a short duration of hyperthermia to induce heat shock proteins expression and enrich the mRNA transcript level of these inducible forms of HSPs for the subsequent molecular cloning work.

Both the nucleotide and translated amino acid sequences of CHO HSP27 (SEQ ID NO: 1 and SEQ ID NO: 2) and HSP70 (SEQ ID NO: 3 and SEQ ID NO: 4) that are obtained showed high similarity to their respective human, mouse and rat homologues. Among these three species, mouse nucleotide and amino acid sequences of both HSPs consistently show the highest homology to the CHO sequences over that of either human or rat:

**Table E3. Homologies with SEQ ID NO: 1 - CHO HSP27 Nucleotide Sequence**

| | |
|---|---|
| Homology to Human HS27 (NM_001540) | 86% |
| Homology to Mouse HSP27 (NM_013560) | 98% |
| Homology to Rat HSP27 (NM_031970) | 92% |

**Table E4. Homologies with SEQ ID NO: 2 - CHO HSP27 Amino Acid Sequence**

| | |
|---|---|
| Homology to Human HSP27 (NP_001531.1) | 90% |
| Homology to Mouse HSP27 (NP_038588.1) | 98% |
| Homology to Rat HSP27 (NP_114176.1) | 98% |

**Table E5. Homologies with SEQ ID NO: 3 - CHO HSP70 Nucleotide Sequence**

| | |
|---|---|
| Homology to Human HSP70 (NM_005345) | 90% |
| Homology to Mouse HSP70 (NM_010479) | 98% |
| Homology to Rat HSP70 (NM_031971) | 95% |

**Table E6. Homologies with SEQ ID NO: 4 - CHO HSP70 Amino Acid Sequence**

| | |
|---|---|
| Homology to Human HSP70 (NP_005336) | 88% |
| Homology to Mouse HSP70 (NP_034609) | 89% |
| Homology to Rat HSP70 (NP_114177) | 89% |

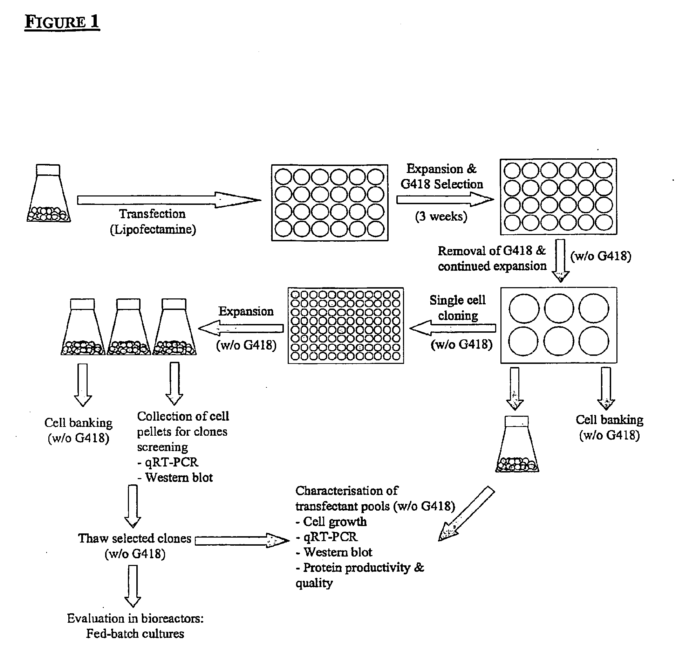

Figure 1 shows the apoptotic pathways regulated by HSP27 and HSP70. It is believed that overexpression of HSP27 and HSP70 in CHO-K1 cells confers on the cells the ability to tolerate adverse growth conditions by targeting multiple points in the cellular apoptotic pathways.

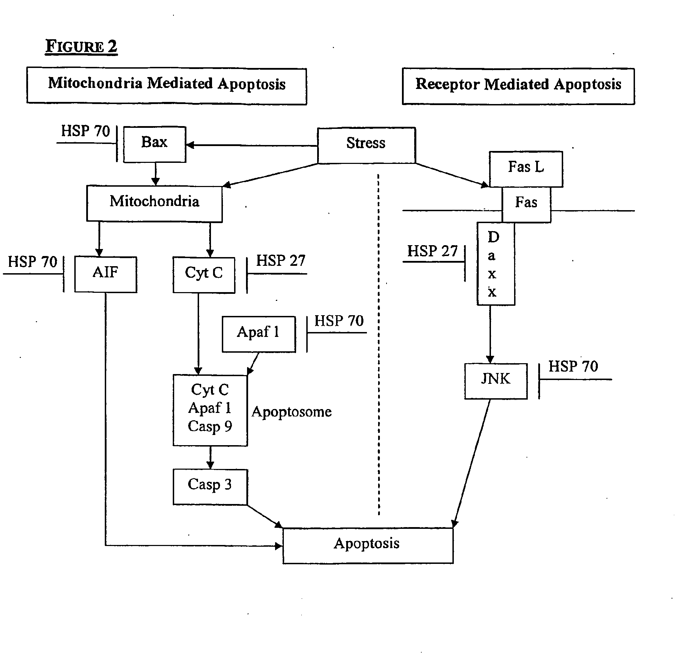

Figure 2 shows an overview of the cellular engineering strategy described in detail in the following Examples.

### Example 3. Construction of Plasmid Vectors for Heat Shock Protein Overexpression

Primers are designed for additional PCR reactions to amplify the sequence verified CHO HSP27 and CHO HSP70 from their respective TOPO cloning vectors (TOPO-CHO-HSP27 and TOPO-CHO-HSP70) and add appropriate restriction enzyme sites to the ends of both sequences at the same time (Table E2).

**Table E2. Primers for addition of restriction sites to HSP27 and HSP70 cloning fragments.**

| **Gene** | **Cloning Fragment** | **Template** | **Forward Primer** | | **Reverse Primer** | |
|---|---|---|---|---|---|---|
| | | | **Restriction Site** | **5' - 3' sequence** | **Restriction Site** | **5' - 3' sequence** |
| HSP27 | HSP27-1 | TOPO-CHO- HSP27 | *Xho*I | CGCCTCGAGGCC ACCATGACCGA | *Mlu*I | GGCCACGCGTA GTGTGCTG |
| HSP70 | HSP70-1 | TOPO- CHO- | *Nhe*I | CAGGCTAGCCGC CATGGCCAAG | *Mlu*I | CCGGACGCGTT CTAATCCAC |
| | HSP70-2 | | *Xba*I | GCGTCTAGAGCC GCCATGGCCAAG | *Sal*I | GCGGTCGACGC AGAGGCCTCT |

The amplified DNA fragments are separated by electrophoresis and purified from agarose gels then subcloned into pCR-TOP02.1 vector using the TOPO-TA cloning kit according to manufacturer's instructions (Invitrogen) to obtain TOPO-CHO-HSP27-1, TOPO-CHO-HSP70-1 and TOPO-CHO-HSP70-2. The HSP27-1 fragment from TOPO-CHO-HSP27-1 is digested and then inserted into the *Xho*I and *Mlu*I multiple cloning site A of the mammalian expression vector, pIRES (BD Biosciences), generating pIRES-27 (Figure 3A).

The HSP70-1 fragment from TOPO-CHO-HSP70-1 is digested and then inserted into the *Nhe*I and *Mlu*I multiple cloning site A of pIRES, generating pIRES-70 (Figure 3B). The HSP70-2 fragment from TOPO-CHO-HSP70-2 is digested and then inserted into the *Xba*I and *Sal*I multiple cloning site B of pIRES-27, generating pIRES-27/70 (Figure 3C). The fragment inserts are sequence verified as described above.

### Example 4. Cell Culture

CHO-IFN-γ is a suspension Chinese Hamster Ovary cell line that expresses recombinant human interferon gamma (Scahill *et al.* 1983). These cells are maintained in commercially available protein-free HyQ CHO MPS medium (Hyclone) supplemented with 4 mM glutamine, 20 mM glucose and 0.25 µM methotrexate (Sigma).

Cells are grown in shaker flasks maintained at 37°C with 8% CO₂ atmosphere and subcultured every 3-4 days.

### Example 5. Detection of Heat Shock Proteins by Western Blot or qRT-PCR

Transfected and non-transfected CHO-IFN-γ cells, on the third day of growth, are pelleted by centrifugation, washed once with ice-cold PBS, and lysed on ice in RIPA buffer containing 50 mM Tris-HCl pH 7.4, 150 mM NaCl, 1% Trition X-100, 1% sodium deoxycholate, 0.1 % SDS, 0.2 mM PMSF, 1 ug/ml pepstatin, 10 ug/ml leupeptin and 1 ug/ml Aprotine. The cell lysates are centrifuged at 14,000 rpm for 30 min at 4°C. The total protein of the supernatants is quantified using BCA Protein Assay kit (Pierce 23225).

The protein samples (25 ug/lane) are treated with LDS sample buffer (NuPAGE NP0007), denatured at 95°C for 5 mins, separated by 4-12% Bis-Tris Gel (NuPAGE NP032) and transferred onto a nitrocellulose membrane. HSP27, HSP70 and Actin are probed with anti-HSP27 (Upstate 06-517, 1:4000 dilution), anti-HSP70 (SPA810F, 1:500 dilution) and anti-Actin (ab8226, 1:4000 dilution) respectively.

The primary antibodies are detected by using anti-rabbit-HRP (ab6721, 1:5000 dilution) or anti-mouse-HRP (ab6728, 1:5000 dilution), and visualized using the ECL HRP chemiluminescent substrate (Amersham RPN 2101) and captured on Lumi-Film Chemiluminescence (Roche).

### Example 6. Generation of Stable Cell Lines

CHO-IFN-γ cells are transfected with the pIRES-27, pIRES-70 and pIRES-27/70 plasmid vectors using Lipofectamin 2000 reagent as per manufacturer's instructions (Invitrogen), to obtain the stable cell lines p27 (overexpress HSP27), p70 (overexpress HSP70) and p27/70 (overexpress both HSP27 and HSP70). Control cell line (pBlank) is generated through transfection of the cells with the plasmid vector backbone.

Geneticin, G418 (Sigma-Aldrich), is added at a concentration of 700 µg/ml and drug resistant cells are collected after 14 days for single cell cloning, in which cells are diluted, and seeded in 96-well plates at one cell per well. Wells that contained more than 1 cell are marked and excluded from further investigation.

50% of the medium in each well is replaced twice a week until surviving clones reached confluence and are expanded for banking and further characterization. Single cell clones are selected based on HSP expression level as detected using Western blots.

### Example 7. Bioreactor Set-Up and Fed-Batch Operations

The instrumentation, reactor set-up and fed-batch operation are previously described (Lee *et al*., 2003). The protein-free feed for the fed-batch cultures are formulated based on a fortified 10X DMEM/F12 (Hyclone) with additional supplementation of 10 g/l soybean protein hydrolysate, HySoy (Quest International).

### Example 8. Determination of Cell Growth and Viability

The cell density in the culture is determined by cell counting using an Improved Neubauer haemocytometer (Weber, England). Cell viability is estimated using the trypan blue exclusion method.

### Example 9. Quantification of Recombinant Human Interferon-γ (IFN-γ)

IFN-γ concentrations in the cultures are determined from serially diluted supernatant samples using an enzyme-linked immunosorbent (ELISA) assay kit (HyCult Biotechnology) as per manufacturer's instructions.

### Example 10. Determination of Caspase 2, 3, 8 and 9 Proteolytic Activities

Cell sample pellets of 2 X 10⁶ cells are collected at 24 hours intervals throughout the cultures. A fluorimetric assay is performed to quantify the caspase 2, 3, 8 and 9 activities in the cells using the BD ApoAlertTM Caspase Assay kit according to manufacturer's instructions (BD Biosciences).

The fluorescence is monitored at an excitation wavelength of 360 nm and an emission wavelength of 465 nm using a fluorescence spectrophotometer, SPECTRAFluor PLUS (Tecan). Raw fluorescence readings are background subtracted and normalized against the start of the culture.

### Example 11. Verification of Heat Shock Protein Overexpression in Transfectants

Cell samples are taken from the late exponential and stationary phases of the fed-batch cultures for western blot analyses. This is to ensure that the clones selected for the bioreactor runs are still over-expressing their respective heat shock proteins in the stages of the cultures where their antiapoptotic capabilities are most essential.

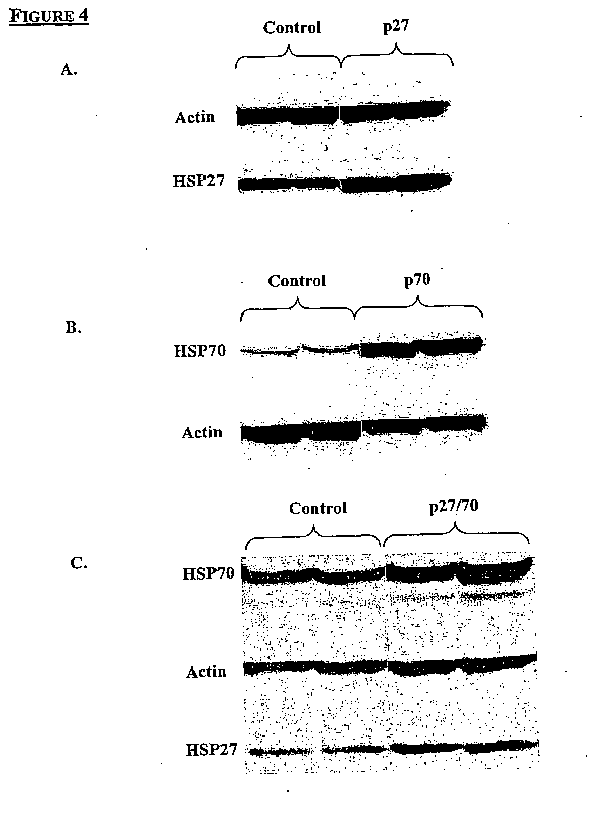

These results confirm significant over-expression of HSP27 in the p27 clone (Figure 4A) and HSP70 in the p70 clone (Figure 4B). Western blot results also verified the over-expression of both HSP27 and HSP70 in the p27/70 clone over the control cells, ie. pBlank (Figure 4C). The higher expression of both HSP27 and HSP70 in the transfectants are visually discernable from the Western blots despite the presence of endogenous levels of each HSP in the Control cells.

The Western blots are also probed for actin and results indicated uniform protein loading for the different samples (Figure 4A, Figure 4B and Figure 4C), thus conclusively showing that the tranfectants expresses higher levels of the respective HSPs than control (pBlank).

### Example 12. Heat Shock Protein Overexpression Delays Apoptosis and Slows Rate of Viability Loss in Bioreactor Fed-Batch Cultures

Fed-batch cultures are used to characterize and evaluate the cell lines since it is the predominant mode of culture process in industry and this enhances the industrial relevance of our study. Additionally, by characterizing the clones in the absence of stresses imposed by depletion of nutrient, we are potentially addressing issues of viability loss that cannot be easily alleviated by conventional media or fed-batch process improvements.

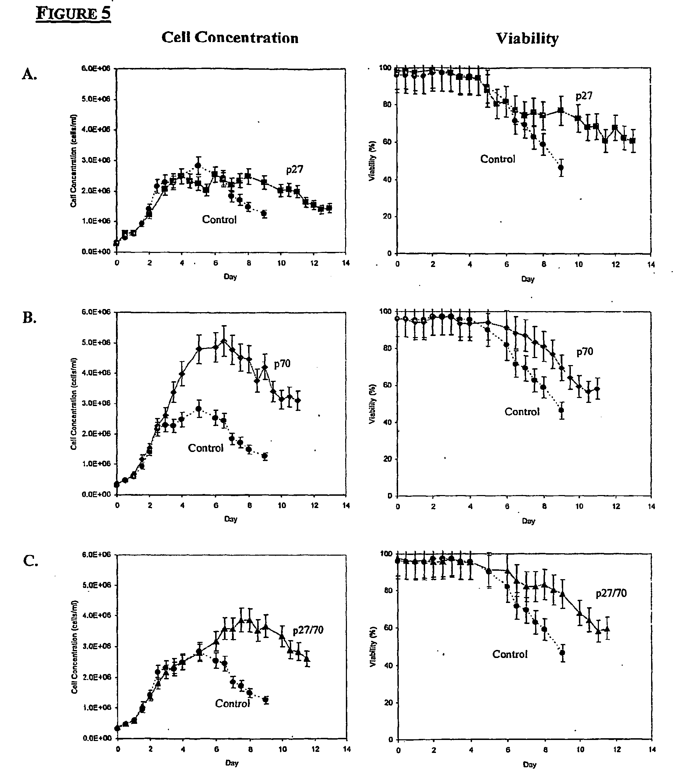

Fed-batch cultures of p27, p70 and p27/70 cells are conducted and compared against Control (pBlank) fed-batch culture. All three engineered cell lines exhibited better culture viability and extensions in culture duration when compared to the Control cells (Figure 5). Compared to the Control culture, p27 exhibited the longest culture time above 50% viability but showed no improvement in maximum cell concentration (Figure 5A).

On the other hand, p70 exhibited the highest maximum cell concentration of almost 2-folds over the Control but also the shortest extension in culture time among the three engineered cell lines (Figure 5B). The p27/70 cell line exhibited improvements in both cell growth and culture duration that is intermediate between the p27 and p70 (Figure 5C).

Caspase assays are performed to confirm that the improvements in culture durations and decrease in rate of viability loss in the three engineered cell lines are indeed due to a delay in the initiation of apoptotic cell death. Caspases 3, 8, 9 and 2 activities are profiled using samples collected from different phases throughout the fed-batch cultures and normalized against the start of each culture.

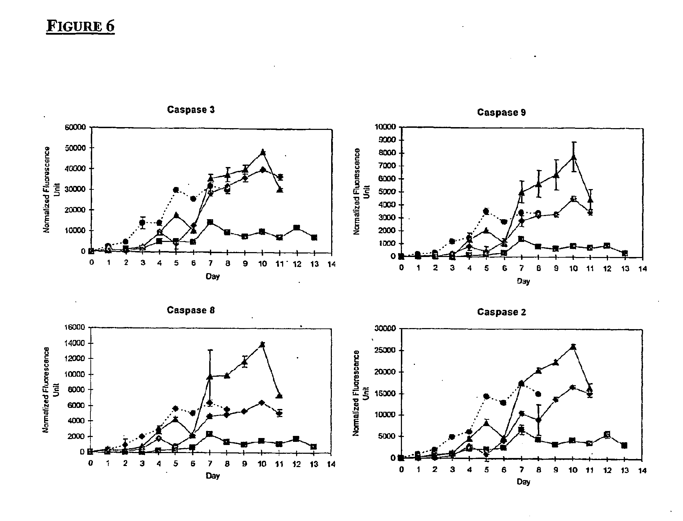

The results indicate that the elevation in caspases activities are delayed by between 24 to 48 hours in the HSP cell lines compared to the Control and corroborates the observation of delayed cell death in the HSP cultures (Figure 6). Furthermore, the much lower level of caspases activities in the p27 cell line probably resulted in the slower loss of viability and hence longer culture duration compared to the Control, p70 and p27/70 cultures.

### Example 13. Heat Shock Protein Overexpression Improves Recombinant Protein Production in Bioreactor Fed-Batch Cultures

Production of the recombinant model protein, human interferon-γ, is used as a means to assess the production capability of the engineered cell lines. Integrated viable cell densities (IVCD) of each culture are calculated from the viable cell concentrations and interferon-γ concentrations are determined from supematent samples collected at regular intervals throughout the cultures.

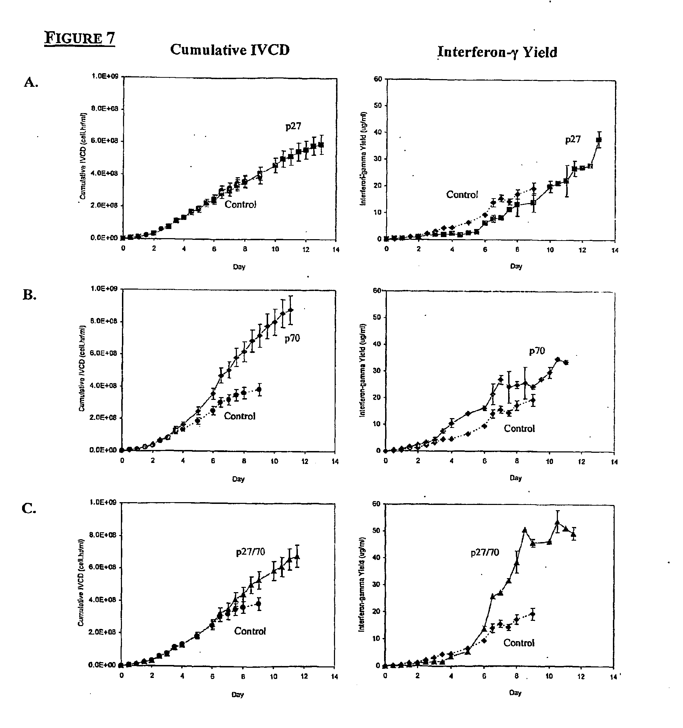

All three engineered cell lines show improved cumulative IVCD over Control of 54%, 129% and 77% for p27, p70 and p27/70 respectively (Figure 7). Improvement in p27 IVCD is solely due to extension in culture lifespan as evident from the complete superimposition of the cumulative IVCD curves with the Control (Figure 7A).

Conversely, improvement in p70 IVCD is primarily due to improvement in growth and maximum cell concentration attained since the cumulative IVCD curves deviate significantly from Control (Figure 7B). p27/70 IVCD improvement, on the other hand, appears to be a combination of significant contributions from each of the factors discussed above (Figure 7C).

Interferon-γ yield from all three engineered cell lines are higher than that for the Control (Figure 7). Despite the difference in cumulative IVCD, both p27 and p70 showed similar interferon-γ yields of 38 and 35 ug/ml respectively, representing 2-folds and 1.8-folds improvements over Control (Figure 7A and Figure 7B).

This suggests that the higher growth in the p70 cultures might have deprived the cells of some of the nutritional resources for production of the recombinant interferon-γ. The combinatorial overexpression of both HSPs in the p27/70 cells resulted in the best yield improvement over Control of 2.8-folds reaching a titer of 54 ug/ml (Figure 7C).

This is attributed to a combination of moderately improved growth and viability extension resulting in longer culture duration at higher cell concentration without compromising production due to competition from exceptionally high cell growth.

### Example 14. Conclusions

The results have shown that overexpression of HSP27 and HSP70, either individually or in combination, can delay the onset of apoptosis in CHO cell cultures and thus extend culture lifespan and improve recombinant protein production. The delays in apoptosis are confirmed by corresponding delays in caspases activation.

This suggests that targeting multiple points in the apoptotic network using heat shock proteins is a feasible and effective cellular engineering strategy. This strategy can find generic application for development of stable robust cell lines with enhanced apoptosis-resistance and hence improved improving culture performance for increase recombinant protein production.

### Example 15. Over-Expression of HSP27 and HSP70 in a CHO Cell Line Expressing Monoclonal Antibody for Improved Culture Viability and Productivity

The objective of this example is to increase monoclonal antibody production in a monoclonal antibody CHO cell line (CHO-mAb). This is achieved via over-expression of HSP27 and HSP70 to improve culture viability and thus productivity.

The CHO-mAb cells is a CHO cell line constructed to express a humanized IgG1 monoclonal antibody against Rhesus(D)-antigen. The purpose for construction of the cell line is for use as a model monoclonal antibody cell line for in-house development work.

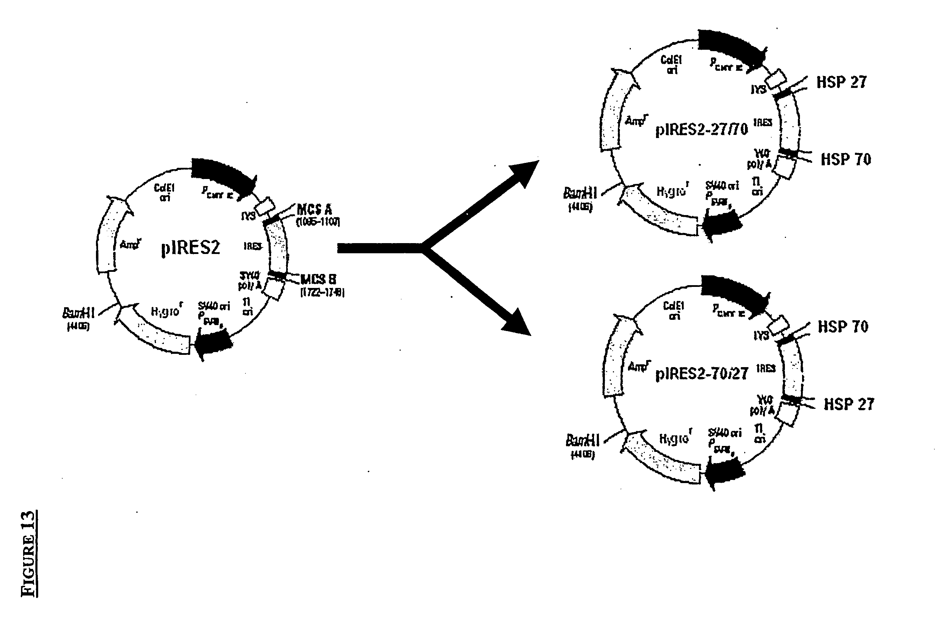

Bicistronic expression vectors are constructed to enable over-expression of both HSP27 and HSP70 in the CHO-mAb cell lines, as shown in Figure 13.

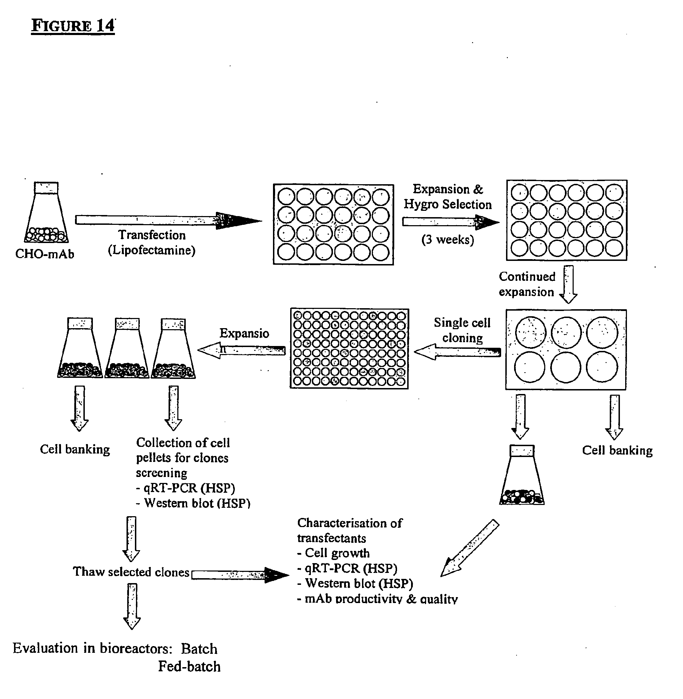

The experimental plan is shown in Figure 14.

The CHO-mAb cell line is transfected with the vectors shown in Figure 13 and antibiotic selected using hygromycin. A negative control transfecting just the vector backbone is also done.

Only transfected cells with plasmids carrying the hygromycin-resistance gene, together with the HSP27 and HSP70 expression cassette, stably integrated into the host genome will survive in the antibiotic. These transfected cell pools are characterized for HSP27 and HSP70 expression as well as improvements in culture viability and productivity.

The transfected cell pools are single-cell cloned to achieve homogenous populations of cell clones with stable over-expression of HSP27 and HSP70. These homogenous single-cell derived populations facilitate better characterization of these cells and final evaluation in the bioreactor systems.

### REFERENCES

Ahmad S., Ahuja R., Venner T. J. and Gupta R. S. (1990). Identification of a protein altered in mutants resistant to microtubule inhibitors as a member of the major heat shock (hsp70) family. Molecular and Cellular Biology, 10(10), 5160-5165.
Arden N. and Betenbaugh M. J. (2004). Life and death in mammalian cell culture: strategies for apoptosis inhibition. TRENDS in Biotechnology, 22(4), 174-180.
Arden N. and Betenbaugh M. J. (2004). Life and death in mammalian cell culture: strategies for apoptosis inhibition. TRENDS in Biotechnology, 22(4), 174-180.
Beere, H. M., Wolf, B. B., Cain, K., Mosser, D. D., Mahboubi, A., Kuwana, T., Tailor, P., Morimoto, R. I., Cohen, G. M. and Green, D. R. (2000) Heat shock protein inhibits apoptosis by preventing recruitment of procaspase 9 to the Apaf 1 apoptosome. Nat Cell Biol. 2, 469-475
Bruey, J. M., Ducasse, C., Bonniaud, P., Ravagnan, L., Susin, S. A., Latoud, C. D., Gurbuxani, S., Arrigo, A. P., Kroemer, G., Solary E. and Garrido, C. (2000) Hsp27 negatively regulates cell death by interacting with cytochrome c. Nat Cell Biol. 2, 645-652.
Charette, S. J., Lavoie, J. N., Lambert, H. and Landry, J. (2000) Inhibition of Daxx-mediated apoptosis by heat shock protein 27. Mol Cell Biol. 20, 7602-7612.
Coss R. A., Sedar A. W., Sistrun S. S., Storck C. W., Wang P. H. and Wachsberger P. R. (2002). Hsp27 protects the cytoskeleton and nucleus from the effects of 42oC at pH 6.7 in CHO cells adapted to growth at pH 6.7. Int. J. Hyperthermia, 18(3), 216-232.
Franklin T. B., Krueger-Naug A. M., Clarke D. B., Arrigo A. P. and Currie R. W. (2005). The role of heat shock proteins Hsp70 and Hsp27 in cellular protection of the central nervousw system. Int. J. Hyperthermia, 21(5), 379-392.
Gabai, V. L., Meriin, A. B., Yaglon, J. A., Wei, J. Y., Mosser, D.D. and Sherman, M. Y. (2000) Suppression of stress kinase JNK is involved in HSP72-mediated protection of myogenic cells from transient energy deprivation. J. Biol Chem. 275, 38088-38094.
Garrido, C., Bruey, J. M., Fromentin, A., Hammann, A., Arrigo, A. P. and Solary, E. (1999) HSP27 inhibits cytochrome c-dependent activation of procaspase-9. FASEB J. 13, 2061-2070.
Garrido, C., Bruey, J. M., Fromentin, A., Hammann, A., Arrigo, A. P. and Solary, E. (1999) HSP27 inhibits cytochrome c-dependent activation of procaspase-9. FASEB J. 13, 2061-2070.
Garrido, C., Gurbuxani, S., Ravagnan, L. and Kroemer, G. (2001) Heat shock proteins: endogenous modulators of apoptotic cell death. Biochem Biophys Res Commun. 286, 433-442.
Gurbuxani S., Schmitt E., Cande C., Parcellier A., Hammann A., Daugas E., Kouranti I., Spahr C., Pance A., Kroemer G. and Garrido C. (2003). Heat shock protein 70 binding inhibits the nuclear import of apoptosis-inducing factor. Oncogene, 22, 6669-6678.
Hargis M. T., Storck C. W., Wickstrom E., Yakubov L. A., Leeper D. B. and Coss R. A. (2004). Hsp27 anti-sense oligonucleotides sensitize the microtubular cytoskeleton of Chinese hamster ovary cells grown at low pH to 42oC-induced reorganization. Int. J. Hyperthermia, 20(5), 491-502.
Ifandi V. and Al-Rubeai M. (2005). Regulation of cell proliferation and apoptosis in CHO-K1 cells by the coexpression of c-Myc and Bcl-2. Biotechnol. Prog., 21, 671-677.
Lasunskaia E. B., Fridlianskaia I. I., Darieva Z. A., da Silva M. S. R., Kanashiro M. M. and Margulis B. A. (2003). Transfection of NS0 myeloma fusion partner cells with HSP70 gene results in higher hybridoma yield by improving cellular resistance to apoptosis. Biotechnology and Bioengineering, 81(4), 496-504
Latchman D. S. (2005). HSP27 and cell survival in neurons. Int. J. Hyperthermia, 21 (5), 393-402.
Mehlen, P., Osthoff, K. S. and Arrigo, A. P. (1996) Small stress protein as novel regulators of apoptosis: heat shock protein 27 blocks Fas/Apo-1 and staurosporine-induced cell death. J. Biol Chem. 271, 16510-16514.
Mosser D. D. and Massie B. (1994). Genetically engineering mammalian cell lines for increased viability and productivity. Biotechnol. Adv., 12(2), 253-277.
Pandey, P., Farber, R., Nakazawa, A., Kumar, S., Bhartu, A., Nalin, C., Weichselbaum, R., Kufe, D. and Kharbanda, S. (2000) Hsp27 functions as a negative regulator of cytochrome c-dependent activation of procaspase 3. Oncogene. 19, 1975-1981.
Patent # US 6451597 B2: Methods for Enhanced Protein Stabilization and for Production of Cell Lines Useful for Production of Such Stabilized Proteins
Patent # US 6468777 B2: Methods for Enhanced Protein Stabilization and for Production of Cell Lines Useful for Production of Such Stabilized Proteins
Patent # US 6495360 B1: Methods for Enhanced Protein Stabilization and for Production of Cell Lines Useful for Production of Such Stabilized Proteins
Patent # US 6541223 B2: Methods for Enhanced Protein Stabilization and for Production of Cell Lines Useful for Production of Such Stabilized Proteins
Patent Application Pub. # US 2005/0048608 A1: Use of Molecular Chaperones for the Enhanced Production of Secreted, Recombinant Proteins in Mammalian Cells
Ravagnan, L., Gurbuxani, S., Susin, S. A., Maisse, C., Daugas, E., Zamzami, N., Mak, T., Jaattela, M., Penninger, J. M., Garrido, C. and Kroemer, G. (2001) Heat shock protein 70 antagonizes apoptosis-inducing factor. Nat Cell Biol. 3, 839-843.
Saleh, A., Srinivasula, S. M., Balkir, L., Robbins, P. D. and Alnemri, E. (2000) Negative regulation of the Apaf-1 apoptosome by HSP70. Nat cell Biol. 2, 476-483.
Singh B., Soltys B. J., Wu Z.-C., Patel H. V., Freeman K. B. and Gupta R. S. (1997). Cloning and some novel characteristics of mitochondrial Hsp70 from Chinese Hamster Cells. Experimental Cell Research, 234, 205-216.
Stankiewicz A. R., Lachapelle G., Foo C. P. Z., Radicioni S. M. and Mosser D. D. (2005). Hsp70 inhibits heat-induced apoptosis upstream of mitochondria by preventing Bax translocation. The Journal of Biological Chemistry, 280(46), 38729-38739.
Vives J., Juanola S., Cairo J. J. and Godia F. (2003). Metabolic engineering of apoptosis in cultured animal cells: implications for the biotechnology industry. Metabolic Engineering, 5, 124-132.
Wlaschin K. F., Nissom P. M., Gatti M. d. L., Ong P. F., Arleen S., Tan K. S., Rink A., Cham B., Wong K., Yap M. and Hu W. S. (2005). EST sequencing for gene discovery in Chinese Hamster Ovary cells. Biotechnology and Bioengineering, 91(5), 592-606.
Wong C. F. D., Wong T. K. K., Heng C. K. and Yap M. G. S. (2006). Targeting early apoptotic genes in batch and fed-batch CHO cells cultures. Biotechnol. Bioeng., In press.

### SEQUENCE LISTINGS

### SEQ ID NO: 1

The 631 bp CHO HSP27 nucleotide sequence

1 ATGACCGAGCGCCGCGTGCCCTTCTCGCTGCTGCGGAGCCCGAGCTGGGAACCATTCCGG
61 GACTGGTACCCTGCACACAGCCGCCTCTTCGATCAAGCTTTCGGGGTGCCCCGGTTGCCC
121 GATGAGTGGTCGCAGTGGTTCAGCGCCGCTGGGTGGCCCGGATACGTGCGCCCGCTGCCC
181 GCCGCGACCGCCGAGGGCCCCGCGGCGGTGACCCTGGCCGCACCAGCCTTCAGCCGAGCG
241 CTCAACCGACAGCTCAGCAGCGGGGTCTCGGAGATCCGACAGACGGCTGATCGCTGGCGC
301 GTGTCCCTGGACGTCAACCACTTCGCTCCGGAGGAGCTCACAGTGAAGACCAAGGAAGGC
361 GTGGTGGAGATCACTGGCAAGCACGAAGAAAGGCAGGACGAACATGGCTACATCTCTCGG
421 TGCTTCACCCGGAAATACACGCTCCCTCCAGGTGTGGACCCCACCCTAGTGTCCTCTTCC
481 CTATCCCCTGAGGGCACACTTACCGTGGAGGCTCCGTTGCCCAAAGCAGTCACGCAGTCG
541 GCGGAGATCACCATTCCGGTTACTTTCGAGGCCCGCGCCCAAATTGGGGGCCCAGAAGCT
601 GGGAAAGTCTGAACAGTCTGGAGCCAAGTAA

| | |
|---|---|
| Homology to Human HS27 (NM_001540) : | 86% |
| Homology to Mouse HSP27 (NM_013560) : | 98% |
| Homology to Rat HSP27 (NM_031970) : | 92% |

### SEQ ID NO: 2

The translated CHO HSP27 amino acid sequence comprising 203 amino acids

1 M T E R R V P F S L L R S P S W E P F R
21 D W Y P A H S R L F D Q A F G V P R L P
41 D E W S Q W F S A A G W P G Y V R P L P
61 A A T A E G P A A V T L A A P A F S R A
81 L N R Q L S S G V S E I R Q T A D R W R
101 V S L D V N H F A P E E L T V K T K E G
121 V V E I T G K H E E R Q D E H G Y I S R
141 C F T R K Y T L P P G V D P T L V S S S
161 L S P E G T L T V E A P L P K A V T Q S
181 A E I T I P V T F E A R A Q I G G P E A
201 G K V

| | |
|---|---|
| Homology to Human HSP27 (NP_001531.1) : | 90% |
| Homology to Mouse HSP27 (NP_038588.1) : | 98% |
| Homology to Rat HSP27 (NP_114176.1) : | 98% |

### SEQ ID NO: 3

The 1749 bp CHO HSP70 nucleotide sequence

1 ATGGCCAAGAACACGGCGATCGGCATCGACCTGGGCACCACCTACTCGTGCGTGGGCGTG
61 TTCCAGCACGGCAAGGTGGAGATCATCGCCAACGACCAGGGCAACCGCACGACCCCCAGC
121 TACGTGGCCTTCACCGACACCGAGCGCCTCATCGGAGACGCCGCCAAGAACCAGGTGGCG
181 CTGAACCCGCAGAACACCGTGTTCGACGCGAAGCGGCTGATCGGCCGCAAGTTCGGCGAT
241 GCGGTGGTGCAGTCCGACATGAAGCACTGGCCCTTCCAGGTGGTGAACGACGGCGACAAG
301 CCCAAGGTGCAGGTGAACTACAAGGGCGAGAGTCGGTCGTTCTTCCCGGAGGAGATCTCG
361 TCCATGGTGCTGACGAAGATGAAGGAGATCGCTGAGGCGTACCTGGGCCACCCGGTGACC
421 AACGCGGTGATCACCGTGCCCGCCTACTTCAACGACTCTCAGCGGCAGGCCACCAAGGAC
481 GCGGGCGTGATCGCCGGTCTAAACGTGCTGCGGATCATCAACGAGCCCACTGCGGCCGCC
541 ATCGCCTACGGGCTGGACCGGAACCGGCAAGGGAAGAAGGACATCAGCCAGAACAAGCTC
601 GCGGTGCGGCGGCTGCGCACTGCGTGTGAGAGGGCCAAGAGGACGCTGTCGTCCAGCACC
661 CAGGCCAGCCTGGAGATCGACTCTCTGTTCGAGGGCATCGACTTCTACACATCCATCACG
721 CGGGCGCGGTTCGAAGAGCTGTGCTCGGACCTGTTCCGCAGCACGCTGGAGCCCGTGGAG
781 AAGGCCCTGCGCGACGCCAAGATGGACAAAGCGCAGATCCACGACCTGGTGCTGGTGGGC
841 GGCTCGACGCGCATCCCCAAGGTGCAGAAGCTGCTGCAGGACTTCTTCAACGGGCGCGAC
901 CTGAACAAGAGCATCAACCCGGACGAGGCGGTGGCCTACGGGGCGGCGGTGCAGGCGGCC
961 ATCCTGATGGGGGACAAGTCGGAGAACGTGCAGGACCTGCTGCTGGACGTGGCGCCG
1021 CTGTCACTGGGCCTGGAGACTGCGGGCGGCGTGATGACGGCGCTCATCAAGCGCAACTCC
1081 ACCATCCCCACCAAGCAGACGCAGACCTTCACCACCTACTCAGACAACCAGCCCGGGGTG
1141 CTGATCCAGGTGTACGAGGGCGAGAGGGCCATGACAGGCGACAACAACCTGCTGGGCGC
1201 TTCGAGCTGAGCGGCATCCCGCCCGCGCCCAGGGGCGTGCCGCAGATCGAGGTGACCTTC
1261 GACATCGACGCCAACGGCCTGAACGTCACGGCCACCGACAAGAGCACCGGCAAGGCCAAC
1321 AAGATCACCATCACCAACGACAAGGGCCGCCTGAGCAAGGAGGAGATCGAGCGCATGGTG
1381 CAGGAGGCCGAGCGCTACAAGGCCGAGGACGAGGTGCAGCGCGACAGGGTGGCCGCCAAG
1441 AACGCGCTCGAATCCTATGCCTTCAACATGAAGAGCGCCGTGGAGGGCGAGGGTCTCAAG
1501 GGCAAGCTCAGCGAGGCTGACAAAAAGAAGGTGCTGGACAAGTGCCAGGAGGTCATCTCC
1561 TGGCTGGACTCCAACACGCTGGCCGATAAGGAGGAGTTCGTGCACAAGCGGGAGGAGCTG
1621 GAGCGGGTGTGCAGCCCCATCATCAGCGGGCTGTACCAGGGTGCGGGTGCTCCTGGGGCT
1681 GGGGGCTTCGGGGCCCAGGCGCCGAAAGGAGCCTCCGGCTCAGGACCCACCATCGAGGAG
1741 GTGGATTAG

| | |
|---|---|
| Homology to Human HSP70 (NM_005345) : | 90% |
| Homology to Mouse HSP70 (NM_010479) : | 98% |
| Homology to Rat HSP70 (NM_031971) : | 95% |

### SEQ ID NO: 4

The translated CHO HSP70 amino acid sequence comprising of 582 amino acids

1 M A K N T A I G I D L G T T Y S C V G V
21 F Q H G K V E I I A N D Q G N R T T P S
41 Y V A F T D T E R L I G D A A K N Q V A
61 L N P Q N T V F D A K R L I G R K F G D
81 A V V Q S D M K H W P F Q V V N D G D K
101 P K V Q V N Y K G E S R S F F P E E I S

121 S M V L T K M K E I A E A Y L G H P V T
141 N A V I T V P A Y F N D S Q R Q A T K D
161 A G V I A G L N V L R I I N E P T A A A
181 I A Y G L D R N R Q G K K D I S Q N K L
201 A V R R L R T A C E R A K R T L S S S T
221 Q A S L E I D S L F E G I D F Y T S I T
241 R A R F E E L C S D L F R S T L E P V E
261 K A L R D A K M D K A Q I H D L V L V G
281 G S T R I P K V Q K L L Q D F F N G R D
301 L N K S I N P D E A V A Y G A A V Q A A
321 I L M G D K S E N V Q D L L L L D V A P
341 L S L G L E T A G G V M T A L I K R N S
361 T I P T K Q T Q T F T T Y S D N Q P G V
381 L I Q V Y E G E R A M T G D N N L L G R
401 F E L S G I P P A P R G V P Q I E V T F
421 D I D A N G L N V T A T D K S T G K A N
441 K I T I T N D K G R L S K E E I E R M V
461 Q E A E R Y K A E D E V Q R D R V A A K
481 N A L E S Y A F N M K S A V E G E G L K
501 G K L S E A D K K K V L D K C Q E V I S
521 W L D S N T L A D K E E F V H K R E E L
541 E R V C S P I I S G L Y Q G A G A P G A
561 G G F G A Q A P K G A S G S G P T I E E
581 V D

| | |
|---|---|
| Homology to Human HSP70 (NP_005336) : | 88% |
| Homology to Mouse HSP70 (NP_034609) : | 89% |
| Horology to Rat HSP70 (NP_114177) : | 89% |

## Claims

1. A method of culturing a Chinese Hamster Ovary (CHO) cell in a bioreactor, the method comprising fed-batch culture of a CHO cell which is engineered, or which an ancestor thereof has been engineered, to up-regulate the expression of HSP27 comprising the sequence set out as SEQ ID NO: 2 or a sequence having at least 98.5% sequence identity thereto, in which up-regulation of expression of HSP27 results in an increase in integrated viable cell density (IVCD) of at least 50%, compared to culture of CHO cells which have not been so engineered.

2. A method according to Claim 1, in which the method comprises introducing an expression vector capable of expressing HSP27, such as pIRES-27 (Figure 3A), into the CHO cell or an ancestor thereof.

3. A method according to Claim 1 or 2, in which the CHO cell is a stable cell line capable of over-expressing HSP27.

4. A method according to any preceding claim, in which the CHO cell displays any one or more of the following: (a) an extension of culture time, such as by between 36 and 72 hours; (b) a delayed or reduced apoptosis; (c) a higher viability; (d) a slower rate of viability loss; (e) a reduced or delayed (such as by between 24 to 48 hours) expression of an apoptotic marker, such as caspase 2, caspase 3, caspase 8 or caspase 9; (f) an increase , in integrated viable cell density (IVCD) of at least 75%, at least 100% or at least 120%; (g) increased maximum cell concentration in culture, or an extension in culture lifespan, or both; (h) enhanced recombinant protein expression, such as enhanced glycoprotein such as interferon-γ (IFN-γ) expression, such as by about 1.8x, about 2x, or about 2.8x; and (i) a higher viability; compared to a CHO cell in which the HSP27 and optionally HSP70 protein(s) is or are not up-regulated.

5. A method according to any preceding claim, in which the CHO cell or ancestor thereof is engineered to up-regulate the expression of HSP70 comprising the sequence SEQ ID NO: 4 or a sequence having at least 90% sequence identity thereto.

6. A method according to Claim 5, in which the method comprises introducing an expression vector capable of expressing HSP70, such as pIRES-70 (Figure 3B) or pIRES-27/70 (Figure 3C) into the CHO cell or an ancestor thereof.

7. A method according to any preceding claim, in which the CHO cell comprises an expression sequence capable of expressing a protein of interest, such as a glycoprotein such as interferon-γ (IFN-γ) or an antibody such as a monoclonal antibody, for example, a humanized IgG1 monoclonal antibody against Rhesus(D)-antigen.

8. A method of expressing a recombinant protein comprising a method according to any preceding claim and expressing a recombinant protein from the CHO cell.

9. A method according to Claim 8, in which the recombinant protein comprises a glycoprotein such as interferon-γ (IFN-γ) or an antibody such as a monoclonal antibody, for example, a humanized IgG1 monoclonal antibody against Rhesus(D)-antigen.

10. A method according to any preceding claim for providing a fed-batch culture of Chinese Hamster Ovary (CHO) cells with extended lifespan in a bioreactor.

11. A method of enhancing expression of Interferon-γ (IFN-γ) or an antibody such as a monoclonal antibody, for example, a humanized IgG1 monoclonal antibody against Rhesus(D)-antigen, by a Chinese Hamster Ovary (CHO) cell in culture, such as by an increase of yield of about 1.8x, about 2x, or about 2.8x, the method comprising a method according to any of Claims 1 to 6.

## Patentansprüche

1. Ein Verfahren zum Kultivieren einer Ovarialzelle eines chinesischen Hamsters (CHO) in einem Bioreaktor, wobei das Verfahren die Fed-Batch-Kultur einer CHO Zelle, die gentechnisch verändert ist oder deren Vorläufer gentechnisch verändert wurde, umfasst, um die Expression von HSP27, welches die Sequenz die als SEQ ID NO:2 dargelegt ist, oder eine Sequenz, die mindestens 98,5 % Sequenzidentität dazu besitzt, hochzuregulieren, wobei die Hochregulation der Expression von HSP27 zu einer Erhöhung der integrierten Dichte lebensfähiger Zellen (IVCD) von mindestens 50 % verglichen mit der Kultur von CHO Zellen, die nicht derart gentechnisch verändert wurden, führt.

2. Ein Verfahren gemäß Anspruch 1, wobei das Verfahren das Einführen eines Expressionssektors, der in der Lage ist, HSP27 zu exprimieren, wie z. B. pIRES-27 (Fig. 3A), in die CHO Zelle oder einen Vorläufer davon umfasst.

3. Ein Verfahren gemäß Anspruch 1 oder 2, wobei die CHO Zelle eine stabile Zelllinie ist, die in der Lage ist, HSP27 zu überexprimieren.

4. Ein Verfahren gemäß einem der vorangehenden Ansprüche, in welchem die CHO-Zelle eines oder mehrere der folgenden zeigt: (a) eine Verlängerung der Kulturzeit, wie z. B. um zwischen 36 und 72 h; (b) eine verzögerte oder reduzierte Apoptose; (c) eine höhere Lebensfähigkeit; (d) eine langsamere Rate des Verlusts an Lebensfähigkeit; (e) eine verringerte oder verzögerte (wie z. B. um zwischen 24 bis 48 h) Expression eines apoptotischen Markers, wie z. B. Caspase 2, Caspase 3, Caspase 8 oder Caspase 9; (f) eine Erhöhung der integrierten Dichte lebensfähiger Zellen (IVCD) um mindestens 75 %, mindestens 100 % oder mindestens 120 %; (g) eine erhöhte maximale Zellkonzentration in der Kultur oder eine Verlängerung in der Lebensspanne der Kultur oder beides; (h) eine erhöhte rekombinante Proteinexpression, wie z. B. erhöhte Glycoprotein, wie z. B. Interferon-γ (IFN- γ)-Expression, wie z. B. um ungefähr 1,8x, ungefähr 2x oder ungefähr 2,8x; und (i) eine höhere Lebensfähigkeit verglichen zu einer CHO Zelle, in der das HSP27 und gegebenenfalls das HSP70 Protein nicht hochreguliert ist.

5. Ein Verfahren gemäß einem der vorangehenden Ansprüche, in welchem die CHO-Zelle oder der Vorläufer davon gentechnisch verändert ist, um die Expression von HSP70 umfassend die Sequenz SEQ ID NO:4 oder eine Sequenz, die mindestens 90 % Sequenzidentität dazu besitzt, hochzuregulieren.

6. Ein Verfahren gemäß Anspruch 5, in welchem das Verfahren das Einführen eines Expressionsvektors, der in der Lage ist, HSP70 zu exprimieren, wie z. B. pIRES-70 (Fig. 3B) oder pIRES-27/70 (Fig. 3C), in die CHO Zelle oder einen Vorläufer davon umfasst.

7. Ein Verfahren gemäß einem der vorangehenden Ansprüche, in dem die CHO Zelle eine Expressionssequenz umfasst, die in der Lage ist, ein Protein von Interesse, wie z. B. ein Glycoprotein, wie z. B. Interferon- γ (IFN- γ) oder einen Antikörper, wie z. B. einen monoklonalen Antikörper, z. B. einen humanisierten IgG1 monoklonalen Antikörper gegen Rhesus(D)-Antigen, zu exprimieren.

8. Ein Verfahren zur Expression eines rekombinanten Proteins umfassend ein Verfahren gemäß einem der vorangehenden Ansprüche und Exprimieren eines rekombinanten Proteins aus der CHO Zelle.

9. Ein Verfahren gemäß Anspruch 8, in welchem das rekombinante Protein ein Glycoprotein umfasst, wie z. B. Interferon- γ (IFN- γ) oder einen Antikörper, wie z. B. einen monoklonalen Antikörper, z. B. einen humanisierten IgG1 monoklonalen Antikörper gegen das Rhesus(D)-Antigen.

10. Ein Verfahren gemäß einem der vorangehenden Ansprüche zum Bereitstellen einer Fed-Batch-Kultur von Ovarialzellen des chinesischen Hamsters (CHO) mit einer verlängerten Lebensspanne in einem Bioreaktor.

11. Ein Verfahren zur Verstärkung der Expression von Interferon- γ (IFN- γ) oder einem Antikörper, wie z. B. einem monoklonalen Antikörper, z. B. einem humanisiertem IgG1 monoklonalen Antikörper gegen das Rhesus(D)-Antigen, durch eine Ovarialzelle eines chinesischen Hamsters (CHO) in Kultur, wie z. B. eine Steigerung der Ausbeute von ungefähr 1,8x, ungefähr 2x oder ungefähr 2,8x, wobei das Verfahren ein Verfahren gemäß einem der Ansprüche 1 bis 6 umfasst.

## Revendications

1. Procédé pour cultiver une cellule d'ovaire de hamster chinois (CHO) dans un bioréacteur, le procédé comprenant une culture en mode fed-batch (culture discontinue alimentée) d'une cellule CHO qui est modifiée, ou dont un ancêtre de celle-ci a été modifié, pour positivement réguler l'expression de HSP27 comprenant la séquence décrite en tant que SEQ ID NO: 2 ou une séquence ayant au moins 98,5 % d'homologie de séquence avec celle-ci, où la régulation positive de l'expression de HSP27 entraîne une augmentation de l'intégrale de la densité des cellules viables (IVCD) d'au moins 50 % par comparaison à une culture de cellules CHO qui n'ont pas été ainsi modifiées.

2. Procédé selon la revendication 1, où le procédé consiste à introduire un vecteur d'expression capable d'exprimer HSP27, tel que pIRES-27 (Figure 3A), dans la cellule CHO ou un ancêtre de celle-ci.

3. Procédé selon la revendication 1 ou 2, dans lequel la cellule CHO est une lignée cellulaire stable capable de surexprimer HSP27.

4. Procédé selon l'une quelconque des revendications précédentes, dans lequel la cellule CHO présente une ou plusieurs des propriétés suivantes : (a) une prolongation de la durée de la culture, tel que de 36 à 72 heures ; (b) une apoptose réduite ou retardée ; (c) une meilleure viabilité ; (d) un taux de perte de viabilité plus lent ; (e) une expression réduite ou retardée (tel que de 24 à 48 heures) d'un marqueur apoptotique, comme la caspase 2, la caspase 3, la caspase 8 ou la caspase 9 ; (f) une augmentation de l'intégrale de la densité des cellules viables (IVCD) d'au moins 75 %, d'au moins 100 % ou d'au moins 120 % ; (g) une concentration cellulaire maximale augmentée en culture, ou une prolongation de la durée de vie de la culture, ou les deux ; (h) une expression améliorée d'une protéine recombinante, tel que l'expression améliorée d'une glycoprotéine comme l'interféron-γ (IFN-γ), tel que d'environ 1,8x, environ 2x, ou environ 2,8x ; et (i) une meilleure viabilité ; par comparaison à une cellule CHO dans laquelle la/les protéine(s) HSP27 et facultativement HSP70 n'est pas ou ne sont pas positivement régulée(s).

5. Procédé selon l'une quelconque des revendications précédentes, dans lequel la cellule CHO ou un ancêtre de celle-ci est modifié(e) pour positivement réguler l'expression de HSP70 comprenant la séquence SEQ ID NO: 4 ou une séquence ayant au moins 90 % d'homologie de séquence avec celle-ci.

6. Procédé selon la revendication 5, où le procédé consiste à introduire un vecteur d'expression capable d'exprimer HSP70, tel que pIRES-70 (Figure 3B) ou pIRES-27/70 (Figure 3C) dans la cellule CHO ou un ancêtre de celle-ci.

7. Procédé selon l'une quelconque des revendications précédentes, dans lequel la cellule CHO comprend une séquence d'expression capable d'exprimer une protéine d'intérêt, comme une glycoprotéine tel que l'interféron-γ (IFN-γ) ou un anticorps tel qu'un anticorps monoclonal, par exemple, un anticorps monoclonal IgG1 humanisé dirigé contre l'antigène Rhésus(D).

8. Procédé pour exprimer une protéine recombinante comprenant un procédé selon l'une quelconque des revendications précédentes, et qui consiste à exprimer une protéine recombinante à partir de la cellule CHO.

9. Procédé selon la revendication 8, dans lequel la protéine recombinante comprend une glycoprotéine tel que l'interféron-γ (IFN-γ) ou un anticorps tel qu'un anticorps monoclonal, par exemple, un anticorps monoclonal IgG1 humanisé dirigé contre l'antigène Rhésus(D).

10. Procédé selon l'une quelconque des revendications précédentes, destiné à permettre une culture en mode fed-batch de cellules d'ovaire de hamster chinois (CHO) ayant une durée de vie prolongée dans un bioréacteur.

11. Procédé pour améliorer l'expression de l'interféron-γ (IFN-γ) ou d'un anticorps tel qu'un anticorps monoclonal, par exemple, un anticorps monoclonal IgG1 humanisé dirigé contre l'antigène Rhésus(D), par une cellule d'ovaire de hamster chinois (CHO) en culture, tel que par une augmentation du rendement d'environ 1,8x, environ 2x, ou environ 2,8x, le procédé comprenant un procédé selon l'une quelconque des revendications 1 à 6.
